# EUROPEAN PATENT APPLICATION

(11) **EP 1 527 782 A1**
(43) Date of publication of application: **04.05.2005**
(21) Application number: 04030120.2
(22) Date of filing: 04.12.2000
(51) Int. Cl.: A61K 31/565, A61K 31/57, A61K 9/14, A61K 9/51, A61K 31/585

(54) **Nanoparticulate eplerenone compositions**

(30) Priority: 08.12.1999 US 169658 P; 02.06.2000 US 208981 P
(62) Divisional of application: 00980277.8
(71) Applicant: Pharmacia Corporation, Chicago, IL 60680 (US)
(72) Inventor: Thosar, Shilpa S., Vernon Hills IL 60061 (US); Gokhale, Rajeev D., Libertyville IL 60048 (US); Tolbert, Dwain S., Wadsworth IL 60083 (US); Desai, Subhash, Wilmette IL 60091 (US)
(74) Representative: Tombling, Adrian George

(57) **Abstract**

There is provided a pharmaceutical composition comprising eplerenone in solid particulate form, wherein at least 90% of the eplerenone particles are smaller than about 15 µm, for example about 0.01 to about 1 µm, in diameter. The composition can be adapted for oral administration, for example as a tablet or capsule comprising eplerenone in a unit dosage amount of about 10 to about 1000 mg and one or more excipients.

## Description

### FIELD OF THE INVENTION

The present invention relates to pharmaceutical compositions comprising eplerenone, more particularly nanoparticulate eplerenone, as an active ingredient, methods of treatment comprising administering such compositions to a subject in need thereof, and the use of such compositions in the manufacture of medicaments.

### BACKGROUND OF THE INVENTION

The compound methyl hydrogen 9,11α-epoxy-17α-hydroxy-3-oxopregn-4-ene-7α,21-dicarboxylate, γ-lactone (referred to herein as eplerenone, also known as epoxymexrenone) was first reported in U.S. Patent No. 4,559,332 to Grob & Kalvoda, which discloses a class of 9,11-epoxy steroid compounds and their salts together with processes for preparation of such compounds. Eplerenone is an aldosterone receptor antagonist that can be administered in a therapeutically effective amount where use of an aldosterone receptor antagonist is indicated, such as in treatment of pathological conditions associated with hyperaldosteronism such as hypertension, heart failure including cardiac insufficiency, and cirrhosis of the liver. U.S. Patent No. 4,559,332, incorporated herein by reference, contains general references to formulations such as tablets and capsules for administration of these 9,11-epoxy steroid compounds.

International Patent Publications No. WO 97/21720 and No. WO 98/25948, both incorporated herein by reference, later disclosed additional synthetic processes for preparation of a similar class of 9,11-epoxy steroid compounds and their salts, including eplerenone.

Eplerenone corresponds in structure to Formula I, below:

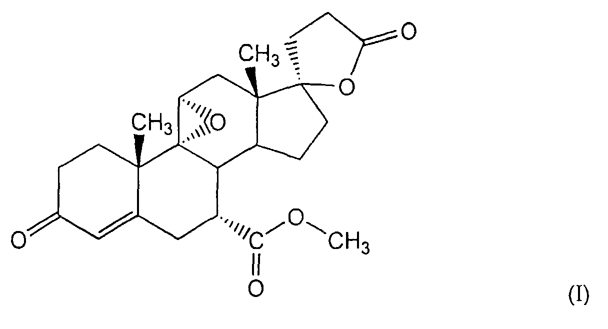

Spironolactone, a 20-spiroxane steroid compound having activity as an aldosterone receptor antagonist, is commercially available for treatment of hypertension. Spironolactone corresponds in structure to Formula II, below:

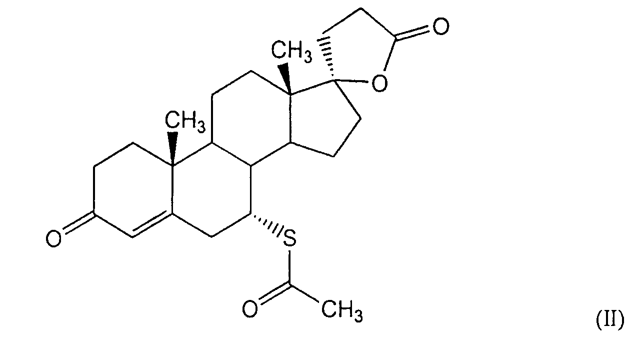

Spironolactone, however, exhibits antiandrogenic activity that can result in gynecomastia and impotence in men, and weak progestational activity that produces menstrual irregularities in women. Commercial formulations sold under the name Aldactone® provide 25, 50 and 100 mg unit doses of spironolactone.

De Gasparo *et al*. (1989), "Antialdosterones: incidence and prevention of sexual side effects", Journal of Steroid Biochemistry 32 (1B), 223-227, report receptor binding studies with spironolactone and eplerenone. Spironolactone having a particle size of 5 µm as commercially formulated, and non-formulated eplerenone having a particle size of 20 µm, were also used in an *in vivo* study of excretion of sodium in urine. Additional *in vivo* and *in vitro* characterization studies of eplerenone are reported by de Gasparo *et al*. (1987), "Three new epoxy-spironolactone derivatives: characterization *in vivo* and *in vitro",* Journal of Pharmacology and Experimental Therapeutics 240, 650-656.

Numerous processes are known and used in the art for preparing drug formulations having primary particle sizes in a desired range, or having a desired mean particle size, or having a particle size distribution characterized by a parameter such as D₉₀, which is defined herein as a linear measure of diameter having a value such that 90% by weight of particles in the formulation, in the longest dimension of the particles, are smaller than that diameter. The terms "nanoparticle" and "nanoparticulate" are used herein to refer to individual particles having a diameter of less than about 15 µm, or to compositions having a D₉₀ particle size of less than about 15 µm. It will be recognized that this is a somewhat broader definition than is commonly used in the art.

According to U.S. Patent No. 5,384,124 to Courteille *et al*., the preparation of microparticles and nanoparticles is principally used to retard dissolution of active principles. On the other hand, U.S. Patent No. 5,145,684 to Liversidge *et al*. discloses nanoparticulate compositions said to provide "unexpectedly high bioavailability" of drugs having low solubility in a liquid medium such as water. International Patent Publication No. WO 93/25190 provides pharmacokinetic data from a rat study indicating a higher apparent rate of absorption from oral administration of a nanoparticulate (average particle size 240-300 nm) than from oral administration of a microparticulate (particle size range 20-30 µm) dispersion ofnaproxen.

Many processes for preparation of nanoparticulate compositions of therapeutic agents are known. Typically, these processes use mechanical means, such as milling, to reduce particle size, or precipitate nanoparticles from solution. Illustrative processes are disclosed in patents and publications listed hereinbelow.

There is a need for development of aldosterone receptor antagonists such as eplerenone that interact minimally with steroid receptors other than aldosterone receptors, for example glucocorticoid, progestin and androgen receptors, and/or that provide for a broader range of treatment options. There is also a need for eplerenone compositions that readily release eplerenone upon oral administration. These and other needs are addressed by the invention hereinbelow described.

### SUMMARY OF THE INVENTION

Now provided is a pharmaceutical composition comprising eplerenone in solid particulate form, wherein the eplerenone has a D₉₀ particle size of less than about 15 µm (herein referred to as "nanoparticulate eplerenone"), preferably less than about 10 µm and more preferably less than about 5 µm, for example about 0.01 to about 1 µm.

Preferably the composition is adapted for oral administration and comprises nanoparticulate eplerenone in a unit dosage amount of about 10 to about 1000 mg.

Oral dosage forms comprising nanoparticulate eplerenone in accordance with the invention can further comprise excipient ingredients. Compositions comprising particular combinations of excipients with nanoparticulate eplerenone are found having improved bioavailability, chemical stability, physical stability, dissolution profile, disintegration time and/or safety, and can have other improved pharmacokinetic, chemical and/or physical properties. Such compositions can exhibit immediate-release or controlled-release behavior, or a combination of both. The present invention is directed not only to such compositions and to unit dosage forms based thereon, but also methods for preparation and use of both.

In a standard dissolution assay using a 1% aqueous sodium dodecyl sulfate dissolution medium, preferred dosage forms of the invention release about 50% of the eplerenone contained therein in 6 hours or less.

### DETAILED DESCRIPTION OF THE INVENTION

Pharmaceutical compositions comprising nanoparticulate eplerenone as the active ingredient in a daily dosage amount of about 10 mg to about 1000 mg according to the present invention exhibit superior performance as aldosterone receptor blockers. These compositions exhibit a high degree of activity, potency, safety and therapeutic effectiveness in such a dosage range. Eplerenone is provided to a subject at a dosage sufficient to provide prolonged blocking of aldosterone receptors and thus confer the desired therapeutic benefit, while maintaining a safe clearance time. Undesirable side effects such as, but not limited to, gastrointestinal irritation, antiandrogenic and progestational activity are also minimized with compositions of the present invention.

Compositions of the invention can, among other pharmacological actions, increase sodium and water excretion with a concomitant potassium-sparing effect. Such compositions can be specifically employed for the prophylaxis and treatment of cardiovascular diseases such as heart failure, hypertension (especially management of mild to moderate hypertension), edema associated with liver insufficiency, post-myocardial infarction, and cirrhosis of the liver. Such compositions can also be used in stroke prevention and in reduction of heart rate for subjects exhibiting an accelerated heart rate. By comparison with known spironolactone compositions, eplerenone compositions of the invention exhibit, among other features, (i) improved selectivity for aldosterone receptors, (ii) reduced binding affinity to the progesterone and androgen receptor, and (iii) reduced interference from plasma proteins.

Besides being useful for human treatment, the present compositions are also useful for veterinary treatment of companion animals, exotic animals and farm animals, particularly mammals including horses, dogs, and cats.

Unformulated eplerenone administered in capsule form is not well absorbed in the gastrointestinal tract. Accordingly, a need exists for suitable oral dosage forms of eplerenone. In one embodiment, the present invention provides such dosage forms that exhibit one or more superior properties relative to unformulated eplerenone and/or other compositions comprising eplerenone. These superior properties include, but are not limited to, one or more of the following:
(1) improved bioavailability;
(2) improved solubility of the pharmaceutical composition;
(3) decreased disintegration time for immediate release oral dosage forms;
(4) decreased dissolution time for immediate release oral dosage forms;
(5) improved dissolution profile for controlled release oral dosage forms;
(6) decreased tablet friability;
(7) increased tablet hardness;
(8) improved safety;
(9) reduced moisture content and/or hygroscopicity;
(10) improved composition wettability;
(11) improved particle size distribution of eplerenone;
(12) improved composition compressibility;
(13) improved composition flow properties;
(14) improved chemical stability of the final oral dosage form;
(15) improved physical stability of the final oral dosage form;
(16) decreased tablet size;
(17) improved blend uniformity;
(18) improved dose uniformity;
(19) increased granule density for wet granulated compositions;
(20) reduced water requirements for wet granulation;
(21) reduced wet granulation time; and/or
(22) reduced drying time for wet granulated mixtures.

### Nanoparticulate eplerenone

It has been discovered that reducing particle size of a solid state form of eplerenone to a D₉₀ particle size (defined elsewhere herein) of about 10 nm to about 15 µm can improve bioavailability of an unformulated or formulated eplerenone composition as compared to an otherwise similar composition having a larger particle size. Accordingly, the D₉₀ particle size of eplerenone particles of the invention or of eplerenone particles present in a composition of the invention is less than about 15 µm, preferably less than about 10 µm, more preferably less than about 1 µm, still more preferably less than about 800 nm, more preferably still less than about 600 nm, and yet more preferably less than about 400 nm.

In one embodiment, the D₉₀ particle size is about 100 nm to about 800 nm, more preferably about 200 nm to about 600 nm. In another embodiment, the D₉₀ particle size is about 400 nm to about 1 µm, more preferably about 500 nm to about 800 nm.

### Treatment of specific conditions and disorders

For treatment of heart failure, a composition of the invention preferably provides a daily dosage of eplerenone in an amount of about 25 mg to about 200 mg, more preferably about 25 mg to about 75 mg, for example about 50 mg. A daily dose of about 0.3 to about 2.7 mg/kg body weight, preferably about 0.3 to about 1 mg/kg body weight, for example about 0.7 mg/kg body weight, can be appropriate.

For treatment of hypertension, a composition of the invention preferably provides a daily dosage of eplerenone in an amount of about 50 mg to about 300 mg, more preferably about 50 mg to about 150 mg, for example about 100 mg. A daily dose of about 0.7 to about 4 mg/kg body weight, preferably about 0.7 to about 2 mg/kg body weight, for example about 1.3 mg/kg body weight, can be appropriate.

For treatment of edema associated with liver insufficiency, a composition of the invention preferably provides a daily dosage of eplerenone in an amount of about 50 mg to about 500 mg, more preferably about 100 mg to about 400 mg, for example about 300 mg. A daily dose of about 0.7 to 6.7 mg/kg body weight, preferably about 1.3 to about 5.3 mg/kg body weight, for example about 4.00 mg/kg body weight, can be appropriate.

In all the above situations, the daily dose can be administered in one to four doses per day, preferably one dose per day. Typically, the present compositions provide a therapeutic effect as aldosterone receptor blockers over a period of about 12 to about 24 hours, preferably a period of about 24 hours, after oral administration.

In general, the present compositions provide a daily dosage of eplerenone sufficient to cause and maintain an average increase of at least about 10% in blood serum renin concentration in humans over a period of about 12 to about 24 hours, preferably a period of about 24 hours, after oral administration. Further, the present compositions generally provide a daily dosage of eplerenone sufficient to cause and maintain an average increase of at least about 50% in blood serum aldosterone concentration in humans over a period of about 12 to about 24 hours, preferably a period of about 24 hours, after oral administration. Still further, the present compositions generally provide a daily dosage of eplerenone sufficient to cause and maintain an average increase in urinary sodium/potassium ratio in humans over a period of about 12 to about 24 hours, preferably a period of about 24 hours, after oral administration. Still further, the present compositions provide a daily dosage of eplerenone sufficient to cause and maintain an average decrease of at least about 5% in diastolic blood pressure in humans over a period of about 12 to about 24 hours, preferably a period of about 24 hours, after oral administration.

### Unit dosages

Compositions of the invention in the form of individual dosage units comprise nanoparticulate eplerenone in an amount of about 10 mg to about 1000 mg, preferably about 20 mg to about 400 mg, more preferably about 25 mg to about 200 mg, and still more preferably about 25 mg to about 150 mg.

Dosage units can typically contain, for example, 10, 20, 25, 37.5, 50, 75, 100, 125, 150, 175, 200, 250, 300, 350 or 400 mg of nanoparticulate eplerenone. Preferred dosage units contain about 25, 50, 100 or 150 mg of nanoparticulate eplerenone. The unit dose can be selected to accommodate any desired frequency of administration used to achieve a specified daily dosage. The dosage regimen (unit dose and frequency) for treating a condition or disorder for which a composition of the invention is useful depends on a variety of factors, including age, weight, sex and medical condition of the subject and severity of the condition or disorder, and thus can vary widely.

Efficacy of the required daily dosage of eplerenone does not appear to materially differ for once-a-day relative to twice-a-day administration with respect to the compositions described herein. While not wishing to be bound by theory, it is hypothesized that compositions of the present invention deliver an amount of eplerenone sufficient to inhibit a protracted response caused by aldosterone binding to the aldosterone receptor site. According to this hypothesis, interruption of aldosterone binding by eplerenone prevents aldosterone-induced gene product synthesis resulting in an extended period of functional aldosterone receptor blockage that does not require a sustained plasma eplerenone concentration. Accordingly, once-a-day administration is generally adequate and is preferred for such tablets for convenience of administration.

### Preparation of eplerenone

The eplerenone of the novel pharmaceutical compositions of the present invention can be prepared by processes known *per se,* including processes set forth in above-cited U.S. Patent No. 4,559,332 and International Patent Publications No. WO 97/21720 and No. WO 98/25948.

### Form of pharmaceutical compositions

In one embodiment, a composition of the present invention comprises nanoparticulate eplerenone and one or more pharmaceutically acceptable carriers, excipients and/or adjuvants (collectively referred to herein as excipients). The excipients are pharmaceutically acceptable in the sense of being compatible with other ingredients of the composition and being non-toxic and otherwise non-deleterious to the recipient. A composition of this embodiment can be adapted for administration by any suitable route, e.g., orally, intravascularly, intraperitoneally, subcutaneously, intramuscularly or rectally, by selection of appropriate excipients and a dosage of eplerenone effective for the treatment intended. For example, these compositions can be prepared in a form suitable for administration. Accordingly, the excipients employed can be solid or liquid, or both, and are preferably formulated with the nanoparticulate eplerenone as a unit-dose composition, for example, a tablet, which can contain about 1% to about 95%, preferably about 10% to about 75%, more preferably about 20% to about 60%, and still more preferably about 20% to about 40%, by weight of nanoparticulate eplerenone. Such pharmaceutical compositions of the invention can be prepared by well known techniques of pharmacy, comprising admixing the components.

### Oral administration

A composition of the invention suitable for oral administration can be prepared, for example, in the form of a tablet, hard or soft capsule, lozenge, pastille, cachet, powder, granules, or suspension, elixir or other liquid. Such a composition is preferably made in the form of a discrete dosage unit containing a predetermined amount of nanoparticulate eplerenone, such as a tablet or capsule. Unit dosage tablets or capsules are preferred.

Pharmaceutical compositions suitable for buccal or sublingual administration include, for example, lozenges comprising nanoparticulate eplerenone in a flavored base, such as sucrose, and acacia or tragacanth, and pastilles comprising nanoparticulate eplerenone in an inert base such as gelatin and glycerin or sucrose and acacia.

Liquid dosage forms for oral administration can include emulsions, solutions, suspensions, syrups, and elixirs containing inert diluents commonly used in the art, such as water. Such liquid dosage forms can also comprise, for example, wetting agents, emulsifying and suspending agents, and sweetening, flavoring and perfuming agents.

Examples of suitable liquid dosage forms include, but are not limited, aqueous solutions comprising nanoparticulate eplerenone and β-cyclodextrin or a water soluble derivative thereof such as sulfobutylether β -cyclodextrin, heptakis-2,6-di-O-methyl-β-cyclodextrin, hydroxypropyl-β-cyclodextrin and dimethyl-β-cyclodextrin.

### Administration by injection

Compositions of the present invention also include formulations suitable for administration by injection, *e.g.*, intravenous, intramuscular, subcutaneous or jet. Such injectable compositions can employ, for example, saline, dextrose or water as a suitable diluent. The pH value of the composition can be adjusted, if necessary, with suitable acid, base, or buffer. Suitable bulking, dispersing, wetting or suspending agents, including mannitol and polyethylene glycol (PEG), *e*.*g*., PEG 400, can also be included in the composition. Nanoparticulate eplerenone can be provided in injection vials. Aqueous diluents can be added to provide a liquid composition suitable for injection.

### Rectal administration

A composition of the invention can be provided in the form of a suppository or the like, suitable for rectal administration. Such rectal formulations preferably contain nanoparticulate eplerenone in a total amount of. for example, about 0.075 to about 30%. preferably about 0.2% to about 20% and most preferably about 0.4% to about 15%. by weight. Carrier excipients such as cocoa butter, theobroma oil, and other oil and PEG suppository bases can be used in such compositions. Other excipients such as coatings (for example, a hydroxypropylmethylcellulose film coating) and disintegrants (for example, croscarmellose sodium and crospovidone) can also be employed if desired.

As indicated above, compositions of the invention can be prepared by any suitable method of pharmacy which includes a step of bringing into association nanoparticulate eplerenone and the desired excipient(s). In general, the compositions are prepared by uniformly and intimately admixing the nanoparticulate eplerenone with a liquid or finely divided excipient, or both, and then, if necessary, shaping the product. For example, a tablet can be prepared by compressing or molding a powder or granules of nanoparticulate eplerenone, optionally with one or more excipients. Compressed tablets can be prepared by compressing, in a suitable machine, the nanoparticulate eplerenone in a free-flowing form, such as powder or granules optionally mixed with a binding agent, lubricant, inert diluent and/or surface-active dispersing agent(s). Molded tablets can be made by molding, in a suitable machine, powdered nanoparticulate eplerenone moistened with an inert liquid diluent.

### Excipients

As noted above, compositions of the invention comprise nanoparticulate eplerenone in a desired amount in combination with one or more pharmaceutically-acceptable excipients appropriate to the desired route of administration. Oral dosage forms of the compositions of the present invention preferably comprise one or more excipients selected from the group consisting of diluents, disintegrants, binding agents and adhesives, wetting agents, lubricants and anti-adherent agents. More preferably, such oral dosage forms are tableted or encapsulated for convenient administration. The resulting tablets or capsules can contain an immediate-release formulation and/or a controlled-release formulation as can be provided, for example, in a dispersion of nanoparticulate eplerenone in hydroxypropylmethylcellulose.

Injectable dosage forms preferably comprise nanoparticulate eplerenone in aqueous or non-aqueous isotonic sterile injection solutions or suspensions. For example, the nanoparticulate eplerenone can be suspended or dissolved in water, polyethylene glycol, propylene glycol, ethanol, corn oil, cottonseed oil, peanut oil, sesame oil, benzyl alcohol, sodium chloride, and/or various buffers. These solutions and suspensions can be prepared from sterile powders or granules having one or more of the excipients mentioned for use in the formulations for oral administration.

Through appropriate selection and combination of excipients, compositions can be provided exhibiting improved performance with respect to, among other properties, efficacy, bioavailability, clearance time, stability, compatibility of the eplerenone with excipients, safety, dissolution profile, disintegration profile and/or other pharmacokinetic, chemical and/or physical properties. The excipients preferably are water soluble or water dispersible and have wetting properties to offset the low aqueous solubility and hydrophobicity of the eplerenone. Where the composition is formulated as a tablet, the combination of excipients selected provides tablets that can exhibit, among other properties, improved dissolution and disintegration profiles, hardness, crushing strength and/or friability.

### Diluents

Compositions of the invention optionally comprise one or more pharmaceutically acceptable diluents as excipients. Suitable diluents illustratively include, either individually or in combination, lactose, including anhydrous lactose and lactose monohydrate; starches, including directly compressible starch and hydrolyzed starches (*e*.*g*., Celutab™ and Emdex™); mannitol; sorbitol; xylitol; dextrose (*e*.*g*., Cerelose™ 2000) and dextrose monohydrate; dibasic calcium phosphate dihydrate; sucrose-based diluents; confectioner's sugar; monobasic calcium sulfate monohydrate; calcium sulfate dihydrate; granular calcium lactate trihydrate; dextrates; inositol; hydrolyzed cereal solids; amylose; celluloses including microcrystalline cellulose, food grade sources of α- and amorphous cellulose (*e*.*g*., Rexcel™) and powdered cellulose; calcium carbonate; glycine; bentonite; polyvinylpyrrolidone; and the like. Such diluents, if present, constitute in total about 5% to about 99%, preferably about 10% to about 85%, and more preferably about 20% to about 80%, of the total weight of the composition. The diluent or diluents selected preferably exhibit suitable flow properties and, where tablets are desired, compressibility.

Lactose and microcrystalline cellulose, either individually or in combination, are preferred diluents. Both diluents are chemically compatible with eplerenone. The use of extragranular microcrystalline cellulose (that is, microcrystalline cellulose added to a wet granulated composition after a drying step) can be used to improve hardness (for tablets) and/or disintegration time. Lactose, especially lactose monohydrate, is particularly preferred. Lactose typically provides compositions having suitable release rates of eplerenone, stability, pre-compression flowability, and/or drying properties at a relatively low diluent cost. It provides a high density substrate that aids densification during granulation (where wet granulation is employed) and therefore improves blend flow properties.

### Disintegrants

Compositions of the invention optionally comprise one or more pharmaceutically acceptable disintegrants as excipients, particularly for tablet formulations. Suitable disintegrants include, either individually or in combination, starches, including sodium starch glycolate (*e*.*g*., Explotab™ of PenWest) and pregelatinized corn starches (*e*.*g*., National™ 1551, National™ 1550, and Colocorn™ 1500), clays (*e*.*g*., Veegum™ HV), celluloses such as purified cellulose, microcrystalline cellulose, methylcellulose, carboxymethylcellulose and sodium carboxymethylcellulose, croscarmellose sodium (*e*.*g*., Ac-Di-Sol™ ofFMC), alginates, crospovidone, and gums such as agar, guar, locust bean, karaya, pectin and tragacanth gums.

Disintegrants may be added at any suitable step during the preparation of the composition, particularly prior to granulation or during a lubrication step prior to compression. Such disintegrants, if present, constitute in total about 0.2% to about 30%, preferably about 0.2% to about 10%, and more preferably about 0.2% to about 5%, of the total weight of the composition.

Croscarmellose sodium is a preferred disintegrant for tablet or capsule disintegration, and, if present, preferably constitutes about 0.2% to about 10%, more preferably about 0.2% to about 7%, and still more preferably about 0.2% to about 5%, of the total weight of the composition. Croscarmellose sodium confers superior intragranular disintegration capabilities to granulated compositions of the present invention.

### Binding agents

Compositions of the invention optionally comprise one or more pharmaceutically acceptable binding agents or adhesives as excipients, particularly for tablet formulations. Such binding agents and adhesives preferably impart sufficient cohesion to the powder being tableted to allow for normal processing operations such as sizing, lubrication, compression and packaging, but still allow the tablet to disintegrate and the composition to be absorbed upon ingestion. Suitable binding agents and adhesives include, either individually or in combination, acacia; tragacanth; sucrose; gelatin; glucose; starches such as, but not limited to, pregelatinized starches (*e*.*g*., National™ 1511 and National™ 1500); celluloses such as, but not limited to, methylcellulose and sodium carboxymethylcellulose (*e*.*g*., Tylose™); alginic acid and salts of alginic acid; magnesium aluminum silicate; polyethylene glycol (PEG); guar gum; polysaccharide acids; bentonites; polyvinylpyrrolidone (povidone or PVP), for example povidone K-15, K-30 and K-29/32; polymethacrylates; hydroxypropylmethylcellulose (HPMC); hydroxypropylcellulose (*e*.*g*., Klucel™); and ethylcellulose (*e*.*g*., Ethocel™). Such binding agents and/or adhesives, if present, constitute in total about 0.5% to about 25%, preferably about 0.75% to about 15%, and more preferably about 1% to about 10%, of the total weight of the composition.

HPMC is a preferred binding agent used to impart cohesive properties to the powder blend of the nanoparticulate eplerenone formulation. HPMC, if present, constitutes in total about 0.5% to about 10%, preferably about 1% to about 8%, and more preferably about 2% to about 4%, of the total weight of the composition. Low molecular weight HPMC having a viscosity of about 2 to about 8 cP typically can be used, although viscosities of about 2 cP to about 6 cP are preferred, particularly viscosities of about 2 cP to about 4 cP. HPMC viscosities are measured as a 2 percent solution in water at 20°C. Methoxy content of the HPMC typically is about 15% to about 35%, whereas hydroxypropyl content is typically up to about 15%, preferably about 2% to about 12%.

### Wetting agents

Eplerenone, even nanoparticulate eplerenone, is largely insoluble in aqueous solution. Accordingly, compositions of the invention optionally but preferably comprise one or more pharmaceutically acceptable wetting agents as excipients. Such wetting agents are preferably selected to maintain the eplerenone in close association with water, a condition that is believed to improve the relative bioavailability of the composition.

Non-limiting examples of surfactants that can be used as wetting agents in compositions of the present invention include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10, and octoxynol 9, poloxamers (polyoxyethylene and polyoxypropylene block copolymers), polyoxyethylene fatty acid glycerides and oils, for example polyoxyethylene (8) caprylic/capric mono- and diglycerides (*e*.*g*., Labrasol™ of Gattefossé), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (*e.g.*, Tween™ 80 of ICI), propylene glycol fatty acid esters, for example propylene glycol laurate (*e.g.*, Lauroglycol™ of Gattefossé), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. Such wetting agents, if present, constitute in total about 0.25% to about 15%, preferably about 0.4% to about 10%, and more preferably about 0.5% to about 5%, of the total weight of the composition.

Wetting agents that are anionic surfactants are preferred. Sodium lauryl sulfate is a particularly preferred wetting agent. Sodium lauryl sulfate, if present, constitutes about 0.25% to about 7%, more preferably about 0.4% to about 4%, and still more preferably about 0.5% to about 2%, of the total weight of the composition.

### Lubricants, glidants and anti-adherents

Compositions of the invention optionally comprise one or more pharmaceutically acceptable lubricants and/or glidants as excipients. Suitable lubricants and/or glidants include, either individually or in combination, glyceryl behapate (*e*.*g*., Compritol™ 888); stearic acid and salts thereof, including magnesium, calcium and sodium stearates; hydrogenated vegetable oils (*e*.*g*., Sterotex™); colloidal silica; talc; waxes; boric acid; sodium benzoate; sodium acetate; sodium fumarate; sodium chloride; DL-leucine; polyethylene glycols (*e*.*g*., Carbowax™ 4000 and Carbowax™ 6000); sodium oleate; sodium lauryl sulfate; and magnesium lauryl sulfate. Such lubricants and/or glidants, if present, constitute in total about 0.1% to about 10%, preferably about 0.2% to about 8%, and more preferably about 0.25% to about 5%, of the total weight of the composition.

Magnesium stearate is a preferred lubricant used, for example, to reduce friction between the equipment and granulated mixture during compression of tablet formulations.

Suitable anti-adherents include talc, comstarch, DL-leucine, sodium lauryl sulfate and metallic stearates. Talc is a preferred anti-adherent or glidant used, for example, to reduce formulation sticking to equipment surfaces and also to reduce static in the blend. Talc, if present, constitutes about 0.1% to about 10%, more preferably about 0.25% to about 5%, and still more preferably about 0.5% to about 2%, of the total weight of the composition.

### Other excipients

Other excipients such as colorants, flavors and sweeteners are known in the pharmaceutical art and can be used in compositions of the present invention. Tablets can be coated, for example with an enteric coating, or uncoated. Compositions of the invention can further comprise, for example, buffering agents.

### Preferred compositions

In one embodiment, a composition of the present invention comprises nanoparticulate eplerenone in a desired amount and one or more cellulosic excipients. The term "cellulosic excipient" embraces excipients comprising cellulose or a derivative thereof, including without restriction purified cellulose, microcrystalline cellulose, and alkylcelluloses and their derivatives and salts (*e.g.*, methylcellulose, ethylcellulose, hydroxypropylcellulose, HPMC, carboxymethylcellulose, sodium carboxymethylcellulose including croscarmellose sodium, *etc*.). Preferably, at least one such cellulosic excipient present is selected from the group consisting of (C₁₋₆ alkyl)celluloses and their derivatives and salts. Still more preferably, this cellulosic excipient is selected from the group consisting of hydroxy(C₂₋₄ alkyl)-(C₁₋₄ alkyl)-celluloses and their derivatives and salts.

Compositions of this embodiment preferably further comprise one or more excipients selected from the group consisting of diluents, disintegrants, binding agents, wetting agents, lubricants and anti-adherent agents. More preferably, these compositions comprise one or more excipients selected from the group consisting of lactose, microcrystalline cellulose, croscarmellose sodium, HPMC, sodium lauryl sulfate, magnesium stearate and talc. Still more preferably, these compositions comprise lactose monohydrate, microcrystalline cellulose, croscarmellose sodium and HPMC, most preferably further comprising one or more additional excipients selected from the group consisting of sodium lauryl sulfate, magnesium stearate and talc.

Individual excipients listed above in the present embodiment optionally can be replaced with other suitable excipients if desired. Acceptable substitute excipients are chemically compatible both with eplerenone and with the other excipients. Although other diluents, disintegrants, binding agents and adhesives, wetting agents, lubricants and/or anti-adherent or glidant agents can be employed, compositions comprising nanoparticulate eplerenone, lactose, microcrystalline cellulose, croscarmellose sodium and HPMC, and, optionally, sodium lauryl sulfate, magnesium stearate and/or talc generally possess a superior combination of phannacokinetic, chemical and/or physical properties relative to such other compositions.

In another embodiment, a composition of the invention comprises:
about 1% to about 95% nanoparticulate eplerenone;
about 5% to about 99% of a pharmaceutically acceptable diluent;
about 0.5% to about 30% of a pharmaceutically acceptable disintegrant; and
about 0.5% to about 25% of a pharmaceutically acceptable binding agent;
all percentages being by weight. Such a composition optionally can additionally comprise about 0.25% to about 15% of a pharmaceutically acceptable wetting agent; about 0.1 % to about 10% of a pharmaceutically acceptable lubricant; and/or about 0.1% to about 15% of a pharmaceutically acceptable anti-adherent agent.

In still another embodiment, a composition of the invention is in the form of an oral unit dosage form, preferably a tablet or capsule, comprising nanoparticulate eplerenone and a cellulosic excipient as defined above. Preferably, the composition comprises one or more excipients selected from the group consisting of lactose monohydrate, microcrystalline cellulose, croscarmellose sodium, hydroxypropyl methylcellulose, sodium lauryl sulfate, magnesium stearate and talc. It is particularly preferred that the various components of such a composition be present in the amounts or weight fractions set forth below.

In an embodiment herein referred to as embodiment A, a composition of the invention is in the form of an oral unit dosage suitable for once-a-day or twice-a-day oral administration and comprises nanoparticulate eplerenone and one or more excipients.

In an embodiment herein referred to as embodiment B, a composition of the invention comprises nanoparticulate eplerenone and one or more excipients and, when orally administered to a human subject in need thereof, provides a therapeutic effect as an aldosterone receptor blocker over a period of about 12 to about 24 hours, preferably a period of at about 24 hours, after administration.

In an embodiment herein referred to as embodiment C, a composition of the invention comprises nanoparticulate eplerenone and one or more excipients and, when orally administered to a human subject in need thereof, causes an average increase of at least about 10% in blood serum renin concentration over a period of about 12 to 24 hours, preferably a period of about 24 hours, after administration.

In an embodiment herein referred to as embodiment D, a composition of the invention comprises nanoparticulate eplerenone and one or more excipients and, when orally administered to a human subject in need thereof, causes an average increase of at least about 50% in blood serum aldosterone concentration over a period of about 12 to 24 hours, preferably a period of about 24 hours, after administration.

In an embodiment herein referred to as embodiment E, a composition of the invention comprises nanoparticulate eplerenone and one or more excipients and, when orally administered to a human subject in need thereof, causes an average decrease of at least about 5% in diastolic blood pressure over a period of about 12 to 24 hours, preferably a period of about 24 hours, after administration.

In an embodiment herein referred to as embodiment F, a composition of the invention comprises nanoparticulate eplerenone and one or more excipients and, when orally administered to a human subject in need thereof, causes an average increase in the urinary sodium/potassium ratio over a period of about 12 to 24 hours, preferably a period of about 24 hours, after administration.

In each of embodiments A-F, the composition preferably comprises nanoparticulate eplerenone and one or more excipients selected from the group consisting of lactose monohydrate, microcrystalline cellulose, croscarmellose sodium, hydroxypropyl methylcellulose, sodium lauryl sulfate, magnesium stearate and talc. It is particularly preferred that the various components of the composition be present in the amounts or weight fractions set forth hereinbelow.

### Immediate-release compositions

Orally deliverable compositions of the present invention include immediate-release compositions and controlled-release compositions. The term "controlled-release" herein refers to compositions exhibiting a sustained or prolonged release profile by comparison with immediate-release compositions.

Preferred immediate-release compositions are in the form of tablets or capsules, especially those comprising nanoparticulate eplerenone in an amount sufficient to provide the desired daily dosage of eplerenone as set forth hereinabove, for example about 50 mg to about 100 mg. Tablets or capsules of different dosage strengths (*e.g.,* 50 mg, 100 mg, *etc*.) can have identical composition and differ only in total size; alternatively, different compositions can be prepared such that the total size of the tablet or capsule is similar for different strengths, by varying the weight fraction of nanoparticulate eplerenone relative to excipients in the formulation.

### Dissolution profile of immediate-release compositions

In a preferred embodiment, immediate-release compositions of the invention exhibit, in an *in vitro* dissolution assay using 1% sodium dodecyl sulfate described hereinbelow, at least about 50% dissolution of the nanoparticulate eplerenone within about 15 minutes. Preferably in this embodiment at least about 80% of the nanoparticulate eplerenone is dissolved *in vitro* within about 30 minutes, and more preferably at least about 90% of the nanoparticulate eplerenone is dissolved *in vitro* within about 45 minutes.

In another preferred embodiment, immediate-release compositions of the invention exhibit, in an *in vitro* dissolution assay using 0.1N hydrochloric acid described hereinbelow, at least about 50% dissolution of the nanoparticulate eplerenone within about 20 minutes. Preferably in this embodiment at least about 80% of the nanoparticulate eplerenone is dissolved *in vitro* within about 45 minutes, more preferably within about 30 minutes, and at least about 90% of the nanoparticulate eplerenone is dissolved *in vitro* within about 90 minutes, more preferably within about 45 minutes.

### Disintegration profile of immediate-release compositions

Excipients for immediate-release compositions of the invention preferably are selected to provide a disintegration time of less than about 30 minutes, preferably less than about 20 minutes, more preferably less than about 18 minutes, and still more preferably less than about 14 minutes, in a standard disintegration assay.

### Granulation particle size and flow properties

Although capsule and tablet compositions of the invention can be prepared, for example, by direct encapsulation or direct compression, they preferably are wet granulated prior to encapsulation or compression. Wet granulation, among other effects, densifies the compositions resulting in improved flow properties, improved compression characteristics and easier metering or weight dispensing of the final compositions. The average particle size of the granulation preferably permits convenient handling and processing and, in the case of tablets, permits formation of a readily compressible mixture that forms pharmaceutically acceptable tablets. The desired tap and bulk densities of the granulation are normally about 0.3 to about 1.0 g/ml, preferably about 0.4 to about 0.8 g/ml.

### Hardness

In preparing tablet formulations, the desired composition is compressed, for example in a conventional production scale tableting machine at normal compression pressure (*e*.*g*., about 1 to about 50 kN). Higher compression pressures produce tablets of greater hardness. Tablet hardness is not critical but is preferably convenient with respect to handling, manufacture, storage and ingestion. Hardness in a range of about 3.5 to about 22 kP is typically acceptable, about 3.5 to about 9 kP being preferred for 25 mg tablets, about 5 to about 13 kP for 50 mg tablets, and about 8 to about 22 kP for 100 mg tablets. The composition should not be compressed to such a degree that there is subsequent difficulty in achieving hydration of the resulting tablet when exposed to gastric fluid.

### Friability

Tablet friability preferably is less than about 0.8%, more preferably less than 0.4%, in a standard friability assay.

### Preferred immediate-release compositions

In a presently preferred embodiment, immediate-release tablets or capsules of the invention comprise:
about 1% to about 90% nanoparticulate eplerenone;
about 5% to about 90% lactose monohydrate;
about 5% to about 90% microcrystalline cellulose; and
about 0.5% to about 10% HPMC;
all percentages being by weight. Such compositions optionally can additionally comprise about 1% to about 10% croscarmellose sodium; about 0.1% to about 7% sodium lauryl sulfate; about 0.1% to about 10% magnesium stearate; and/or about 0.1 % to about 10% talc.

More preferably, immediate-release tablets or capsules of this embodiment comprise:
about 19% to about 40% nanoparticulate eplerenone;
about 32% to about 52% lactose monohydrate;
about 8% to about 28% microcrystalline cellulose;
about 1% to about 10% croscarmellose sodium; and
about 1% to about 8% HPMC;
all percentages being by weight. Such tablets or capsules optionally can additionally comprise about 0.1% to about 7% sodium lauryl sulfate; about 0.1% to about 10% magnesium stearate; and/or about 0.1% to about 10% talc. Preferably the HPMC has a viscosity of about 2 to about 8 cP, more preferably about 2 to about 6 cP. Preferably such compositions are in the form of tablets.

Still more preferably, such tablets comprise:
about 24% to about 35% nanoparticulate eplerenone;
about 37% to about 47% lactose monohydrate;
about 13% to about 23% microcrystalline cellulose;
about 2% to about 6% croscarmellose sodium; and
about 2% to about 4% HPMC;
all percentages being by weight. Such tablets optionally can additionally comprise about 0.25% to about 4% sodium lauryl sulfate; about 0.25% to about 5% magnesium stearate; and about 0.1% to about 5% talc. Preferably, the HPMC has a viscosity of about 2 to about 6 cP.

Still more preferably, such tablets comprise:
about 28% to about 31 % nanoparticulate eplerenone;
about 41 % to about 43% lactose monohydrate;
about 17% to about 19% microcrystalline cellulose;
about 4.5% to about 5.5% croscarmellose sodium; and
about 2.5% to about 3.5% HPMC;
all percentages being by weight. Such tablets optionally can additionally comprise about 0.5% to about 1.5% sodium lauryl sulfate; about 0.25% to about 0.75% magnesium stearate; and about 0.5% to about 1.5% talc. Preferably, the HPMC has a viscosity of about 2 to about 4 cP.

Illustratively, an immediate-release composition of this embodiment is in the form of a coated or uncoated unit dosage tablet that prior to coating comprises:
29.4% nanoparticulate eplerenone;
42.0% lactose monohydrate;
18.1% microcrystalline cellulose;
5.0% croscarmellose sodium;
3.0% HPMC of viscosity 2-4 cP;
1.0% sodium lauryl sulfate;
0.5% magnesium stearate; and
1.0% talc;
all percentages being by weight.

Individual immediate-release tablets or capsules of this embodiment preferably comprise:
about 20 to about 110 mg nanoparticulate eplerenone;
about 30 to about 150 mg lactose monohydrate;
about 10 to about 70 mg microcrystalline cellulose; and
about 1 to about 15 mg HPMC.
Such tablets or capsules optionally can additionally comprise about 1 to about 25 mg croscarmellose sodium; about 0.25 to about 5 mg of sodium lauryl sulfate; about 0.5 to about 3 mg magnesium stearate; and about 0.5 to about 5 mg talc. Preferably, the HPMC has a viscosity of about 2 to about 8 cP, more preferably about 2 to about 6 cP.

Illustrative of this embodiment are individual immediate-releasc tablets or capsules comprising:
about 23 to about 27 mg nanoparticulate eplerenone;
about 34 to about 38 mg lactose monohydrate;
about 14 to about 17 mg microcrystalline cellulose;
about 3 to about 6 mg croscarmellose sodium; and
about 1 to about 4 mg HPMC.
Such tablets or capsules optionally can additionally comprise about 0.25 to about 1.5 mg sodium lauryl sulfate; about 0.1 to about 1 mg magnesium stearate; and about 0.25 to about 1.5 mg talc. Preferably, the HPMC has a viscosity of about 2 to about 6 cP. Preferably such compositions are in the form of tablets.

Also illustrative of this embodiment are individual immediate-release tablets or capsules comprising:
about 48 to about 52 mg nanoparticulate eplerenone;
about 70 to about 73 mg lactose monohydrate;
about 29 to about 33 mg microcrystalline cellulose;
about 6 to about 10 mg croscarmellose sodium; and
about 4 to about 6 mg HPMC.
Such tablets or capsules optionally can additionally comprise about I to about 2.5 mg sodium lauryl sulfate; about 0.5 to about 1.5 mg magnesium stearate; and about 1 to about 2.5 mg talc. Preferably, the HPMC has a viscosity of about 2 to about 6 cP. Preferably such compositions are in the form of tablets.

Also illustrative of this embodiment are individual immediate-release tablets or capsules comprising:
about 98 to about 102 mg nanoparticulate eplerenone;
about 141 to about 145 mg lactose monohydrate;
about 60 to about 64 mg microcrystalline cellulose;
about 16 to about 18 mg croscarmellose sodium; and
about 9 to about 11 mg HPMC.
Such tablets or capsules optionally can additionally comprise about 3 to about 4 mg sodium lauryl sulfate; about 1 to about 2 mg magnesium stearate; and about 3 to about 4 mg talc. Preferably, the HPMC has a viscosity of about 2 to about 6 cP. Preferably such compositions are in the form of tablets.

In another presently preferred embodiment, immediate-release tablets or capsules of the invention comprise:
about 15% to about 35% nanoparticulate eplerenone;
about 48% to about 68% lactose monohydrate; and
about 2% to about 22% microcrystalline cellulose; all percentages being by weight. Such tablets or capsules optionally can additionally comprise about 0.1% to about 10% croscarmellose sodium; about 0.1% to about 7% sodium lauryl sulfate; about 0.1% to about 10% magnesium stearate; about 0.1% to about 10% talc; and about 0.1% to about 10% colloidal silicon dioxide.

More preferably, immediate-release tablets or capsules of this embodiment comprise:
about 20% to about 30% nanoparticulate eplerenone;
about 53% to about 63% lactose monohydrate;
about 6.5% to about 16.5% microcrystalline cellulose; and
about 0.5% to about 6% croscarmellose sodium;
all percentages being by weight. Such tablets or capsules optionally can additionally comprise about 0.25% to about 4% sodium lauryl sulfate; about 0.25% to about 5% magnesium stearate; about 0.5% to about 5% talc; and about 0.1% to about 5% colloidal silicon dioxide. Preferably such compositions are in the form of capsules.

Still more preferably, such capsules comprise:
about 23% to about 27% nanoparticulate eplerenone;
about 56% to about 60% lactose monohydrate;
about 9.5% to about 13.5% microcrystalline cellulose; and
about 0.5% to about 3.5% croscarmellose sodium;.
All percentages being by weight. Such capsules optionally can additionally comprise about 0.25% to about 1.5% sodium lauryl sulfate; about 0.1 % to about 1% magnesium stearate; about 1% to about 4% talc; and about 0.1 % to about 1.5% colloidal silicon dioxide.

Illustratively, an immediate-release composition of this embodiment is in the form of a capsule that comprises:
25.0% nanoparticulate eplerenone;
57.9% lactose monohydrate;
11.3% microcrystalline cellulose;
2.0% croscarmellose sodium;
0.5% sodium lauryl sulfate;
0.3% magnesium stearate;
2.5% talc; and
0.5% colloidal silicon dioxide;
all percentages being by weight.

Individual immediate-release tablets or capsules of this embodiment preferably comprise:
about 20 to about 110 mg nanoparticulate eplerenone;
about 48 to about 242 mg lactose monohydrate; and
about 2 to about 56 mg microcrystalline cellulose.
Such tablets or capsules optionally can additionally comprise about 0.25 to about 18 mg croscarmellose sodium; about 0.1 to about 5 mg sodium lauryl sulfate; about 0.1 to about 5 mg magnesium stearate; about 0.5 to about 8 mg talc; and about 0.1 to about 5 mg colloidal silicon dioxide.

Illustrative of this embodiment are individual immediate-release tablets or capsules comprising:
about 23 to about 27 mg nanoparticulate eplerenone;
about 56 to about 60 mg lactose monohydrate;
about 9.5 to about 13.5 mg microcrystalline cellulose; and
about 0.5 to about 3.5 mg croscarmellose sodium.
Such tablets or capsules optionally can additionally comprise about 0.1 to about 1.5 mg sodium lauryl sulfate; about 0.1 to about 1.5 mg magnesium stearate; about 0.25 to about 4.5 mg talc; and about 0.1 to about 2.5 mg colloidal silicon dioxide. Preferably such compositions are in the form of capsules.

Also illustrative of this embodiment are individual immediate-release tablets or capsules comprising:
about 48 to about 52 mg nanoparticulate eplerenone;
about 114 to about 118 mg lactose monohydrate;
about 21 to about 15 mg microcrystalline cellulose; and
about 2 to about 6 mg croscarmellose sodium.
Such tablets or capsules optionally can additionally comprise about 1 to about 2.5 mg sodium lauryl sulfate; about 0.25 to about 1.5 mg magnesium stearate; about 2 to about 8 mg talc; and about 0.1 to about 3 mg colloidal silicon dioxide. Preferably such compositions are in the form of capsules.

Also illustrative of this embodiment are individual immediate-release tablets or capsules comprising:
about 98 to about 102 mg nanoparticulate eplerenone;
about 229 to about 234 mg lactose monohydrate;
about 43 to about 48 mg microcrystalline cellulose; and
about 6 to about 10 mg croscarmellose sodium.
Such tablets or capsules optionally can additionally comprise about 0.5 to about 4 mg sodium lauryl sulfate; about 0.5 to about 3 mg magnesium stearate; about 8 to about 12 mg talc; and about 0.5 to about 4 mg colloidal silicon dioxide. Preferably such compositions are in the form of capsules.

### Controlled-release compositions

Compositions of the present invention also include controlled-release formulations, including formulations providing prolonged or sustained delivery of the drug to the gastrointestinal tract by any known mechanism. Such mechanisms include, but are not limited to, pH-sensitive release based on the pH of the small intestine; slow erosion of a tablet or of beads contained in a capsule; retention in the stomach based on physical properties of the formulation; bioadhesion of the dosage form to the mucosal lining of the intestinal tract; and enzymatic release of eplerenone from the formulation. The intended effect is to extend the time period over which eplerenone is delivered to the site of action by adaptation of the formulation. Thus, for example, enteric-coated controlled-release formulations of nanoparticulate eplerenone are within the scope of the present invention.

Preferred controlled-release compositions are in the form of tablets or capsules, especially those comprising nanoparticulate eplerenone in an amount sufficient to provide the desired daily dosage of eplerenone as set forth hereinabove, for example about 25 mg to about 100 mg. As in the case of immediate-release compositions, tablets or capsules of different dosage strengths (*e*.*g*., 50 mg, 100 mg, *etc*.) can have identical composition and differ only in total size; alternatively, different compositions can be prepared such that the total size of the tablet or capsule is similar for different strengths, by varying the weight fraction of nanoparticulate eplerenone relative to excipients in the formulation.

One type of controlled-release composition, for example, achieves sustained release by having the nanoparticulate eplerenone held in a matrix formed of a pharmaceutically acceptable matrix-forming material. Suitable matrix-forming materials include without restriction waxes, *e*.*g*., carnauba wax, beeswax, paraffin wax, ceresine, shellac wax, fatty acids and fatty alcohols; oils, hardened oils and fats, *e.g.*, hardened rapeseed oil, castor oil, beef tallow, palm oil and soya bean oil; polymers, *e*.*g*., microcrystalline cellulose, ethylcellulose, hydroxypropylcellulose, povidone, HPMC, PEG, methacrylates, *e*.*g*., polymethylmethacrylate (PMMA), and carbomer; alginates; and xanthan gums.

Other controlled-release compositions achieve sustained release by use of granulates, coated powders, beads, pellets or the like, by use of multi-layering, and/or by use of osmotic pump technology. Suitable delivery systems for providing sustained release can be adapted by those of skill in the art from disclosures in patent literature, including without limitation the patents listed below, each of which is incorporated herein by reference.
U.S. Patent No. 3,362,880 to Jeffries.
U.S. Patent No. 4,316,884 to Alam & Eichel.
U.S. Patent No. 4,601,894 to Hanna & Vadino.
U.S. Patent No. 4,708,861 to Popescu *et al*.
U.S. Patent No. 4,753,802 to Hamel & Stephens.
U.S. Patent No. 4,765,989 to Wong *et al*.
U.S. Patent No. 4,795,641 to Kashdan.
U.S. Patent No. 4,847,093 to Ayer & Wong.
U.S. Patent No. 4,867,985 to Heafield *et al*.
U.S. Patent No. 4,892,778 to Cortese *et al*.
U.S. Patent No. 4,940,588 to Sparks & Geoghegan.
U.S. Patent No. 4,975,284 to Nabahi & Stead.
U.S. Patent No. 5,055,306 to Barry *et al*.
U.S. Patent No. 5,057,317 to Iida.
U.S. Patent No. 5,082,668 to Barclay *et al*.
U.S. Patent No. 5,160,742 to Mazer *et al*.
U.S. Patent No. 5,160,744 to Huynh *et al*.
U.S. Patent No. 5,190,765 to Huynh *et al*.
U.S. Patent No. 5,273,760 to Oshlack *et al*.
U.S. Patent No. 5,292,534 to Valentine & Valentine.
U.S. Patent No. 5,296,236 to Golzi & Santus.
U.S. Patent No. 5,415,871 to Pankhania *et al*.
U.S. Patent No. 5,451,409 to Rencher *et al*.
U.S. Patent No. 5,455,046 to Baichwal.
U.S. Patent No. 5,472,711 to Baichwal.
U.S. Patent No. 5,478,574 to Baichwal & Staniforth.
U.S. Patent No. 5,518,730 to Fuisz.
U.S. Patent No. 5,523,095 to Wilson *et al*.
U.S. Patent No. 5,527,545 to Santus *et al*.
U.S. Patent No. 5,536,505 to Wilson *et al*.
U.S. Patent No. 5,536,508 to Canal *et al*.
U.S. Patent No. 5,571,533 to Bottoni *et al*.
U.S. Patent No. 5,773,025 to Santus *et al*.
U.S. Patent No. 5,858,344 to Müller & Cremer.
U.S. Patent No. 6,093,420 to Baichwal.
European Patent No. 0 572 942.
International Patent Publication No. WO 89/08119.
International Patent Publication No. WO 91/16920.
International Patent Publication No. WO 92/13547.
International Patent Publication No. WO 93/12765.
International Patent Publication No. WO 93/17673.
International Patent Publication No. WO 94/27582.
International Patent Publication No. WO 96/16638.
International Patent Publication No. WO 98/01117.
International Patent Publication No. WO 99/61005.
International Patent Publication No. WO 00/18374.
International Patent Publication No. WO 00/33818.
International Patent Publication No. WO 00/40205.

In controlled-release compositions of the invention that employ one or more coating materials for granules, beads, pellets, tablets, *etc*., suitable coating materials include, but are not limited to, any pharmaceutically acceptable polymer, *e*.*g*., ethylcellulose, cellulose acetate, cellulose acetate butyrate and polymethacrylates containing quaternary ammonium groups, PEG, hydroxypropylcellulose, HPMC, povidone, polyvinyl alcohol and enteric polymers; monomeric materials such as sugars, *e.g.*, lactose, sucrose, fructose and mannitol; salts including sodium chloride, potassium chloride and derivatives thereof; and organic acids, *e.g.*, fumaric acid, succinic acid, lactic acid, tartaric acid and mixtures thereof. Suitable enteric polymers include polyvinyl acetate phthalate, cellulose acetate phthalate, cellulose acetate trimellitate, shellac, zein, and polymethacrylates containing carboxyl groups. These polymers can be applied as solutions or latexes. Other barrier coatings can be used such as waxes.

The coating material or blend of coating materials can be plasticized according to properties of the material or blend such as the glass transition temperature of the main component or mixture of components, or properties of the solvent used for applying the coating. Suitable plasticizers can be added at up to about 50% by weight of the coating composition. Such plasticizers include, for example, diethyl phthalate, citrate esters, PEG, glycerol, acetylated glycerides and castor oil.

Tablets or capsules containing nanoparticulate eplerenone can be coated directly to produce a controlled-release dosage form, or can comprise a plurality of coated cores containing nanoparticulate eplerenone. As used herein, the term "core" refers to an element of the composition containing eplerenone and various carrier. Each core can contain an amount of nanoparticulate eplerenone in the range of about 0.1 % to about 95%, preferably about 10% to about 80%, by weight. The core typically can be about 200 to about 1700 µm in diameter. Standard coating procedures such as those described, for example, in Remington: The Science and Practice of Pharmacy, 19th Edition, Mack Publishing Co. (1995) can conveniently be used.

Controlled-release compositions of the invention can be made by processes known in the art including prilling, spray drying, pan coating, melt granulation, wet or dry granulation, Wurster coating, tangential coating, top spraying, tableting, extruding, coacervation and the like. Particle size of components of these compositions other than nanoparticulate eplerenone depends on the process used, and can range from less than 1 µm to about 500 µm for powder processes (*e*.*g*., mixtures, spray drying, dispersions and the like); about 5 to about 1700 µm for coating processes (Wurster, top spray, bottom spray, spray drying, extrusion, layering and the like); and about 1 to about 20 mm for tableting processes. Except where the controlled-release particle is a whole tablet, the particles are then combined into a single dosage form such that the amount of nanoparticulate eplerenone in the dosage form provides the desired unit dose.

Dual-release compositions, containing nanoparticulate eplerenone in an immediate-release form in association with nanoparticulate eplerenone in a controlled-release form, are a further embodiment of the invention. The immediate-release form of nanoparticulate eplerenone in such compositions typically constitutes about 0.5% to about 90% of the total amount of eplerenone of the composition, with the controlled-release form constituting the remainder of the nanoparticulate eplerenone present in the composition. As a result, the composition provides an amount of nanoparticulate eplerenone for release immediately following administration and an additional amount of nanoparticulate eplerenone for controlled release.

### Illustrative controlled-release capsule having coated beads

In a controlled-release composition of the invention comprising coated beads, otherwise known as pellets, the coated beads can be presented for example in a sachet, capsule or tablet. The following non-limiting example describes a capsule containing coated beads. All percentages are by weight.

A plurality of cores containing nanoparticulate eplerenone are prepared by extrusion and spheronization, or by layering nanoparticulate eplerenone or a blend of nanoparticulate eplerenone with one or more excipients on to particles comprising one or more excipients. The cores themselves can be immediate-release or controlled-release formulations depending on the materials and method of manufacture, and contain about 0.1 % to 100% nanoparticulate eplerenone.

An extruded core having immediate-release properties typically contains nanoparticulate eplerenone and, for example, about 0.5% to about 99.9% of a disintegrant such as microcrystalline cellulose; about 0.5% to about 50% of a binding agent such as hydroxypropylcellulose; about 0.5% to about 90% of a filler or diluent such as lactose; and optionally other excipients. An extruded core can, where desired, consist essentially of nanoparticulate eplerenone and the binding agent.

An extruded core having controlled-release properties typically contains nanoparticulate eplerenone and, for example, about 0.5% to about 50% of a swelling and/or gelling polymer such as hydroxypropylcellulose; or about 10% to about 90% of a hydrophobic material such as cetyl alcohol.

A layered core can contain nanoparticulate eplerenone deposited on an inert carrier such as a sugar sphere, which accounts for about 10% to about 90% of the core, together with about 0.1% to about 50% of a binding agent. The core can further contain one or more diluents, wetting agents and/or other excipients. The binding agent can be selected to provide immediate release (e.g., hydroxypropylcellulose, HPMC and the like) or controlled release (*e.g.*, ethylcellulose, cellulose acetate butyrate and enteric binding materials such as hydroxypropylmethylcellulose phthalate, polyvinyl acetate phthalate and the like).

A portion of the complete dosage form can be immediate-release cores as described above. Such immediate-release cores can be coated with a rapidly disintegrating or dissolving coat for aesthetic, handling or stability purposes. Suitable coating materials for these purposes include povidone, hydroxypropylcellulose, HPMC, PEG and polymethacrylates containing free amino groups. Such materials can further contain plasticizers, antitack agents and/or diluents. Dyes or colorants can also be added to the coating material for aesthetic reasons or to provide a distinctive appearance. An addition of about 3% of the weight of the core as coating material generally provides a continuous coat.

The controlled-release portion of the dosage form can be provided by controlled-release cores as described above, by controlled-release cores further modified by overcoating, or by immediate release cores modified by overcoating.

A typical coating composition for making the controlled-release component contains an insoluble matrix polymer in an amount of about 15% to about 85%, and a water-soluble material in an amount of about 15% to about 85%, by weight of the coating composition. Optionally, an enteric polymer in an amount from about 0.1% to 100% by weight of the coating composition can be used. Suitable insoluble matrix polymers include ethylcellulose, cellulose acetate butyrate, cellulose acetates and polymethacrylates containing quaternary ammonium groups. Suitable water-soluble materials include polymers such as PEG, hydroxypropylcellulose, HPMC, povidone and polyvinyl alcohol; monomeric materials such as sugars (*e.g.*, lactose, sucrose, fructose, mannitol and the like); salts (*e.g.*, sodium chloride, potassium chloride and the like); organic acids (*e.g.*, fumaric acid, succinic acid, lactic acid, tartaric acid and the like); and mixtures thereof. Suitable enteric polymers include hydroxypropylmethylcellulose acetate succinate (HPMCAS), hydroxypropylmethylcellulose phthalate (HPMCP), polyvinyl acetate phthalate, cellulose acetate phthalate, cellulose acetate trimellitate, shellac, zein, polymethacrylates containing carboxyl groups, and the like.

The coating composition can include about 0.1% to 100% of a filler, which can illustratively be silicon dioxide, titanium dioxide, talc, kaolin, alumina, starch, powdered cellulose, microcrystalline cellulose, polacrilin potassium or the like.

The coating composition can be applied to the cores as a solution or latex in one or more organic or aqueous solvents or mixtures thereof. Where solutions are applied, the solvent is present in an amount of about 25% to about 99%, preferably about 85% to about 97%, by weight of the solution. Suitable solvents are water, lower alcohols, lower chlorinated hydrocarbons, ketones and mixtures thereof. Where latexes are applied, the solvent is present in an amount of about 25% to about 97%, preferably about 60% to about 97%, by weight of the latex. The solvent can be predominantly water.

### Illustrative controlled-release matrix tablet

A controlled-release composition of the invention that is a matrix tablet formulation contains nanoparticulate eplerenone together with a swelling and/or gelling polymer such as L-hydroxypropylcellulose admixed with a diluent/disintegrant such as microcrystalline cellulose. The excipients in the tablet formulation can be processed (*e.g.*, spray dried) together, prior to compression to form the tablet. Matrix tablets of this type often exhibit a rapid initial release of the drug until the polymers swell and gel, thereby inducing controlled release for the remainder of the drug.

The quantity of immediate release and duration of controlled release can be varied by altering the quantities of the excipients used. If the immediate-release component is not large enough, a quantity of nanoparticulate eplerenone can be included in a rapidly dissolving outer coat of a polymeric material such as PEG or HPMC.

The following non-limiting example describes a matrix tablet. All percentages are by weight

A typical matrix tablet can contain the swelling and/or gelling polymer in an amount of about 5% to about 70%, and one or more diluents in an amount of about 15% to about 90%. Diluents can be soluble materials such as lactose, mannitol, sorbitol or the like, or insoluble materials such as tribasic calcium phosphate, powdered cellulose or starch.

Additionally, the tablet can contain a lubricant in an amount of about 0.1% to about 8%, selected for example from metal stearates, stearic acid, hydrogenated oils such as soya bean oil or castor oil, sodium stearyl fumarate, polytetrafluoroethylene, talc and the like.

Matrix tablets can be coated for aesthetic, handling or stability purposes, or to increase the quantity of the immediate-release portion of eplerenone. In this latter case, nanoparticulate eplerenone is dissolved or suspended in the coating solution and sprayed on to the tablets until the desired quantity of eplerenone has been added. The coating material can be added to any desired thickness but weight gains in the range of about 1% to about 20% are typical, preferably about 2% to about 10%, and more preferably about 2% to about 5%, for example about 3%. The coating can be applied exactly as described above for coated beads.

Alternatively, the controlled-release component of the nanoparticulate eplerenone can be provided in the form of coated beads and the immediate-release component can be included in the body of the tablet. Such a tablet disintegrates to release the immediate-release component, leaving the coated beads to provide the controlled-release component. Coated beads can be present in an amount of about 1% to about 60%, preferably about 5% to about 50%, and more preferably about 5% to about 40%, by weight of the tablet. Suitable diluents/disintegrants for tablets of this type are microcrystalline cellulose, starches and the like.

Preferably in a matrix tablet, the matrix is hydrophilic and releases eplerenone at a relatively constant rate over a period of up to about 6 hours. This hydrophilic matrix can be prepared, for example, by incorporating HPMC into the formulation in combination with other excipients. The amount and type of HPMC used depends upon the release rate desired.

In preparing a typical matrix tablet of the invention, HPMC is combined with nanoparticulate eplerenone and other excipients, and the resulting blend is then wet granulated under high shear, fluid bed dried, blended and compressed to form a tablet. To provide controlled-release properties, the HPMC preferably is of high molecular weight, having a viscosity (as determined using a 2% aqueous solution at 20°C) of about 3,500 to about 5,600 cP.

When the tablet is exposed to an aqueous medium such as that of the gastrointestinal tract, the tablet surface wets and the matrix polymer begins to partially hydrate forming an outer gel layer. This outer gel layer becomes fully hydrated and begins to erode into the aqueous medium. Water continues to permeate toward the core of the tablet permitting another gel layer to form beneath the eroding outer gel layer. These successive concentric gel layers sustain uniform release of eplerenone by diffusion from the gel layer and exposure through tablet erosion.

In general, increasing the concentration of the polymer in the matrix increases the viscosity of the gel that forms on the tablet surface and causes a decrease in diffusion and release rate of eplerenone. Typical two-hour controlled-release formulations (that is, formulations releasing about 50% of the eplerenone *in vitro* during the two-hour period after ingestion) comprise about 2% to about 20%, preferably about 3% to about 17%, and more preferably about 4% to about 14%, high molecular weight HPMC. Typical four-hour controlled-release formulations (that is, formulations releasing about 50% of the eplerenone *in vitro* during the four-hour period after ingestion) comprise about 5% to about 45%, preferably about 7% to about 35%, and more preferably about 8% to about 28%, high molecular weight HPMC. Typical six-hour controlled-release formulations (that is, formulations releasing about 50% of the eplerenone *in vitro* during the six-hour period after ingestion) comprise about 10% to about 45%, preferably about 12% to about 35%, and more preferably about 14% to about 35%, high molecular weight HPMC.

Dissolution profiles can be further adjusted by appropriate selection of high molecular weight HPMC concentrations. In addition, dissolution time tends to decrease as HPMC particle size increases. This is likely due to poor hydration of the hydroxypropyl methylcellulose matrix as particle size increases. Smaller particle size, on the other hand, can cause rapid hydration of the matrix and therefore slower drug release rate.

Changes in tablet size and shape can affect the surface to volume ratio of the tablet and therefore the drug release kinetics from the hydrophilic matrix. In general, it has been discovered that release of nanoparticulate eplerenone from matrix tablets of the present invention is enhanced when tablet size is decreased and/or tablet shape is changed from a round to a caplet shape. Further, because tablet coating can alter eplerenone release kinetics, the effect of the coating on drug release should be considered for coated tablets. Release of eplerenone from a matrix tablet is substantially independent of tablet compression force in a range of compression forces from about 10 to about 40 kN.

### Preferred controlled-release compositions

In a presently preferred embodiment, controlled-release tablets or capsules of the invention comprise:
about 20% to about 40% nanoparticulate eplerenone;
about 30% to about 50% lactose monohydrate;
about 10% to about 30% microcrystalline cellulose;
about 1% to about 16% high molecular weight HPMC; and
about 0.5% to about 13% low molecular weight HPMC;
all percentages being by weight. Such compositions optionally can additionally comprise about 0.1% to about 10% magnesium stearate and/or about 0.1% to about 10% talc. Preferably, the low molecular weight HPMC has a viscosity of about 2 to about 8 cP, more preferably about 2 to about 6 cP. Preferably, the high molecular weight HPMC has a viscosity value of about 3,500 to about 5,600 cP, as also discussed before. These compositions preferably are in the form of tablets.

More preferably, such tablets comprise:
about 25% to about 35% nanoparticulate eplerenone;
about 35% to about 45% lactose monohydrate;
about 15% to about 25% microcrystalline cellulose;
about 1% to about 11 % high molecular weight HPMC; and
about 0.5% to about 8% low molecular weight HPMC;
all percentages being by weight. Such tablets or capsules optionally can additionally comprise about 0.1 % to about 5.5% magnesium stearate and/or about 0.1% to about 6% talc.

Still more preferably, such tablets comprise:
about 28% to about 32% nanoparticulate eplerenone;
about 38% to about 42% lactose monohydrate;
about 17.5% to about 21.5% microcrystalline cellulose;
about 4% to about 8% high molecular weight HPMC; and
about 2% to about 5% low molecular weight HPMC;
all percentages being by weight. Such tablets optionally can additionally comprise about 0.1% to about 2.5% magnesium stearate and/or about 0.1% to about 3% talc.

In another presently preferred embodiment, controlled-release tablets or capsules of the invention comprise:
about 20% to about 40% nanoparticulate eplerenone;
about 15% to about 47% lactose monohydrate;
about 3.5% to about 28.5% microcrystalline cellulose;
about 1% to about 45% high molecular weight HPMC; and
about 0.5% to about 13% low molecular weight HPMC;
all percentages being by weight. Such compositions optionally can additionally comprise about 0.1 % to about 10% magnesium stearate and/or about 0.1 % to about 10% talc. Preferred viscosities for high and low molecular weight HPMCs are as in the previous embodiment. These compositions preferably are in the form of tablets.

More preferably, such tablets comprise:
about 25% to about 35% nanoparticulate eplerenone;
about 22% to about 42% lactose monohydrate;
about 8.5% to about 23.5% microcrystalline cellulose;
about 5% to about 35% high molecular weight HPMC; and
about 0.5% to about 8% low molecular weight HPMC;
all percentages being by weight. Such tablets or capsules optionally can additionally comprise about 0.1 % to about 5.5% magnesium stearate and/or about 0.1 % to about 6% talc.

Still more preferably, such tablets comprise:
about 28% to about 32% nanoparticulate eplerenone;
about 25% to about 39% lactose monohydrate;
about 11.5% to about 20.5% microcrystalline cellulose;
about 10% to about 35% high molecular weight HPMC; and
about 2% to about 5% low molecular weight HPMC;
all percentages being by weight. Such tablets optionally can additionally comprise about 0.1% to about 2.5% magnesium stearate and/or about 0.1% to about 3% talc.

In another presently preferred embodiment, controlled-release tablets or capsules of the invention comprise:
about 20% to about 40% nanoparticulate eplerenone;
about 20% to about 40% lactose monohydrate;
about 5% to about 25% microcrystalline cellulose;
about 10% to about 30% high molecular weight HPMC; and
about 0.5% to about 13% low molecular weight HPMC;
all percentages being by weight. Such compositions optionally can additionally comprise about 0.1 % to about 10% magnesium stearate and/or about 0.1 % to about 10% talc. Preferred viscosities for high and low molecular weight HPMCs are as in the previous embodiment. These compositions preferably are in the form of tablets.

More preferably, such tablets comprise:
about 25% to about 35% nanoparticulate eplerenone;
about 25% to about 35% lactose monohydrate;
about 10% to about 20% microcrystalline cellulose;
about 15% to about 25% high molecular weight HPMC; and
about 0.5% to about 8% low molecular weight HPMC;
all percentages being by weight. Such tablets or capsules optionally can additionally comprise about 0.1% to about 5.5% magnesium stearate and/or about 0.1% to about 6% talc.

Still more preferably, such tablets comprise:
about 28% to about 32% nanoparticulate eplerenone;
about 28.5% to about 32.5% lactose monohydrate;
about 13% to about 17% microcrystalline cellulose;
about 18% to about 22% high molecular weight HPMC; and
about 2% to about 5% low molecular weight HPMC;
all percentages being by weight. Such tablets optionally can additionally comprise about 0.1 % to about 2.5% magnesium stearate and/or about 0.1 % to about 3% talc.

In another presently preferred embodiment, individual controlled-release tablets or capsules of the invention comprise:
about 25 to about 150 mg nanoparticulate eplerenone;
about 12.5 to about 190 mg lactose monohydrate;
about 2 to about 100 mg microcrystalline cellulose;
about 10 to about 80 mg high molecular weight HPMC; and
about 1 to about 25 mg low molecular weight HPMC.
Such compositions optionally can additionally comprise about 0.1 to about 10 mg magnesium stearate and/or about 0.5 to about 15 mg talc. Preferred viscosities for high and low molecular weight HPMCs are as in the previous embodiment.

In another presently preferred embodiment, individual controlled-release tablets or capsules of the invention comprise:
about 95 to about 105 mg nanoparticulate eplerenone;
about 128 to about 139 mg lactose monohydrate;
about 60 to about 70 mg microcrystalline cellulose;
about 10 to about 25 mg high molecular weight HPMC; and
about 5 to about 15 mg low molecular weight HPMC.
Such compositions optionally can additionally comprise about 0.1 to about 7 mg magnesium stearate and/or about 0.5 to about 8 mg talc. Preferred viscosities for high and low molecular weight HPMCs are as in the previous embodiment. These compositions preferably are in the form of tablets.

More preferably, such tablets comprise:
about 98 to about 102 mg nanoparticulate eplerenone;
about 131 to about 136 mg lactose monohydrate;
about 63 to about 67 mg microcrystalline cellulose;
about 18 to about 22 mg high molecular weight HPMC; and
about 8 to about 12 mg low molecular weight HPMC.
Such tablets optionally can additionally comprise about 0.5 to about 3 mg magnesium stearate and/or about 2 to about 5 mg talc.

In another presently preferred embodiment, individual controlled-release tablets or capsules of the invention comprise:
about 45 to about 55 mg nanoparticulate eplerenone;
about 35 to about 55 mg lactose monohydrate;
about 17.5 to about 27.5 mg microcrystalline cellulose;
about 37 to about 47 mg high molecular weight HPMC; and
about I to about 10 mg low molecular weight HPMC.
Such compositions optionally can additionally comprise about 0.1 to about 6 mg magnesium stearate and/or about 0.5 to about 7 mg talc. Preferred viscosities for high and low molecular weight HPMCs are as in the previous embodiment. These compositions preferably are in the form of tablets.

More preferably, such tablets comprise:
about 48 to about 52 mg nanoparticulate eplerenone;
about 43 to about 47 mg lactose monohydrate;
about 20.5 to about 24.5 mg microcrystalline cellulose;
about 40 to about 44 mg high molecular weight HPMC; and
about 3 to about 7 mg low molecular weight HPMC.
Such tablets optionally can additionally comprise about 0.1 to about 3 mg magnesium stearate and/or about 0.5 to about 3 mg talc.

In another presently preferred embodiment, individual controlled-release tablets or capsules of the invention comprise:
about 95 to about 105 mg nanoparticulate eplerenone;
about 110 to about 130 mg lactose monohydrate;
about 50 to about 70 mg microcrystalline cellulose;
about 30 to about 50 mg high molecular weight HPMC; and
about 5 to about 15 mg low molecular weight HPMC.
Such compositions optionally can additionally comprise about 0.1 to about 7 mg magnesium stearate and/or about 0.5 to about 8 mg talc. Preferred viscosities for high and low molecular weight HPMCs are as in the previous embodiment. These compositions preferably are in the form of tablets.

More preferably, such tablets comprise:
about 98 to about 102 mg nanoparticulate eplerenone;
about 118 to about 122 mg lactose monohydrate;
about 58 to about 62 mg microcrystalline cellulose;
about 38 to about 42 mg high molecular weight HPMC; and
about 8 to about 12 mg low molecular weight HPMC.
Such tablets optionally can additionally comprise about 0.5 to about 3 mg magnesium stearate and/or about 2 to about 5 mg talc.

In another presently preferred embodiment, individual controlled-release tablets or capsules of the invention comprise:
about 145 to about 155 mg nanoparticulate eplerenone;
about 175 to about 195 mg lactose monohydrate;
about 87.5 to about 97.5 mg microcrystalline cellulose;
about 45 to about 55 mg high molecular weight HPMC; and
about 10 to about 20 mg low molecular weight HPMC.
Such compositions optionally can additionally comprise about 0.1 to about 8 mg magnesium stearate and/or about 0.5 to about 10 mg talc. Preferred viscosities for high and low molecular weight HPMCs are as in the previous embodiment. These compositions preferably are in the form of tablets.

More preferably, such tablets comprise:
about 148 to about 152 mg nanoparticulate eplerenone;
about 183 to about 187 mg lactose monohydrate;
about 90 to about 95 mg microcrystalline cellulose;
about 48 to about 52 mg high molecular weight HPMC; and
about 13 to about 17 mg low molecular weight HPMC;
Such tablets optionally can additionally comprise about 0.5 to about 4.5 mg magnesium stearate and/or about 3 to about 7 mg talc.

In another presently preferred embodiment, individual controlled-release tablets or capsules of the invention comprise:
about 95 to about 105 mg nanoparticulate eplerenone;
about 95 to about 110 mg lactose monohydrate;
about 45 to about 55 mg microcrystalline cellulose;
about 60 to about 75 mg high molecular weight HPMC; and
about 5 to about 15 mg low molecular weight HPMC.
Such compositions optionally can additionally comprise about 0.1 to about 7 mg magnesium stearate and/or about 0.5 to about 8 mg talc. Preferred viscosities for high and low molecular weight HPMCs are as in the previous embodiment. These compositions preferably are in the form of tablets.

More preferably, such tablets comprise:
about 98 to about 102 mg nanoparticulate eplerenone;
about 99 to about 104 mg lactose monohydrate;
about 48 to about 52 mg microcrystalline cellulose;
about 64.5 to about 68.5 mg high molecular weight HPMC; and
about 8 to about 12 mg low molecular weight HPMC.
Such tablets optionally can additionally comprise about 0.5 to about 3 mg magnesium stearate and/or about 2 to about 5 mg talc.

In a standard *in vitro* dissolution assay using 1% aqueous sodium dodecyl sulfate as the dissolution medium, controlled-release compositions of one embodiment of the invention release about 50% of the eplerenone contained therein in 3 hours or less, but not less than about 1.5 hours, preferably not less than about 1.75 hours, for example around 2 hours after initiation of the assay.

Controlled-release compositions of another embodiment release in the same assay about 50% of the eplerenone contained therein in 4.5 hours or less, but not less than about 3.5 hours, preferably not less than about 3.75 hours, for example around 4 hours after initiation of the assay.

Controlled-release compositions of still another embodiment release in the same assay about 50% of the eplerenone contained therein in 6 hours or less, but not less than about 5 hours, preferably not less than about 5.5 hours, for example 6 hours after initiation of the assay.

### Alternative epoxyspiroxane compounds

In compositions of the present invention, other 9,11-epoxy-20-spiroxane compounds, particularly 9,11-epoxy-20-spiroxane compounds that are aldosterone antagonists, can be substituted for eplerenone. Such alternative 9,11-epoxy-20-spiroxane compounds can be prepared by processes known *per se,* for example as set forth in above-cited U.S. Patent No. 4,559,332. These compounds include, but are not limited to, the following:
9α,11α-epoxy-7α-methoxycarbonyl-15β,16β-methylene-20-spirox-4-ene-3,21-dione;
9α,11α-epoxy-7α-isopropoxycarbonyl-20-spirox-4-ene-3,21-dione;
9α,11α-epoxy-7α-ethoxycarbonyl-20-spirox-4-ene-3,21-dione;
9α,11α-epoxy-6β,7β-methylene-20-spirox-4-ene-3,21-dione;
9α,11α-epoxy-6β,7β;15β,16β-bis-methylene-20-spirox-4-ene-3,21-dione;
9α,11α-epoxy-17β-hydroxy-6β,7β-methylene-3-oxo-17α-pregn-4-ene-21-carboxylic acid;
9α,11α-epoxy-17β-hydroxy-6β,7β-methylene-3-oxo-17α-pregn-4-ene-21-carboxylic acid methyl ester;
9α,11α-epoxy-17β-hydroxy-6β,7β;15β,16β-bis-methylene-3-oxo-17α-pregn-4-ene-21-carboxylic acid methyl ester;
9α,11α-epoxy-6β,7β-methylene-20-spiroxa-1,4-diene-3,21-dione;
9a, 11α-epoxy-17β-hydroxy-7α-methoxycarbonyl-3-oxo-17α-pregn-4-ene-21-carboxylic acid;
9α,11α-epoxy-17β-hydroxy-3-oxo-17β-pregn-4-ene-7α,21-dicarboxylic acid dimethyl ester;
9α,11α-epoxy-17β-hydroxy-7α-isopropoxycarbonyl-3-oxo-17α-pregn-4-ene-21-carboxylic acid;
9α,11α-epoxy-17β-hydroxy-7α-ethoxycarbonyl-3-oxo-17α-pregn-4-ene-21-carboxylic acid;
9α,11α-epoxy-6α,7α-methylene-20-spirox-4-ene-3,21-dione;
9α,11α-epoxy-17β-hydroxy-3-oxo-17α-pregn-4-ene-7α,21-dicarboxylic acid dimethyl ester; and
9α,11α-epoxy-17β-hydroxy-15β,16β-methylene-3-oxo-17α-pregn-4-ene-7α,21-dicarboxylic acid dimethyl ester;
and pharmaceutically acceptable salts thereof.

### Methods of treatment

The present invention also is directed to therapeutic methods of treating a condition or disorder where treatment with an aldosterone receptor blocker is indicated, such methods comprising oral administration of nanoparticulate eplerenone, preferably in a daily dose of about 10 to about 1000 mg per day, and preferably formulated in a composition as described herein, one or more times to a subject, preferably a human subject, in need thereof. A dosage regimen to prevent, give relief from, or ameliorate the condition or disorder preferably comprises once-a-day or twice-a-day oral administration of a therapeutically or prophylactically effective dose of such a composition, more preferably a 25 mg, 50 mg, 100 mg or 150 mg oral unit dose, but can be modified in accordance with a variety of factors. These factors include the type, age, weight, sex, diet, and medical condition of the subject and the severity of the condition or disorder. Thus, the dosage regimen actually employed can vary widely and therefore deviate from the preferred dosage regimen set forth above.

Initial treatment can begin with dosages indicated above. Treatment is generally continued as necessary over a period of several weeks to several months or years until the condition or disorder has been controlled or eliminated. Patients undergoing treatment with compositions disclosed herein can be routinely monitored by any of the methods well known in the art to determine effectiveness of therapy. Continuous analysis of such data permits modification of the treatment regimen during therapy so that optimally effective amounts of nanoparticulate eplerenone are administered at any point in time, and so that the duration of treatment can be determined as well. In this way, the treatment regimen can be rationally modified over the course of therapy so that the lowest amount of eplerenone exhibiting satisfactory effectiveness is administered, and so that administration is continued only so long as is necessary to successfully treat the condition or disorder.

The present invention further encompasses use of nanoparticulate eplerenone, preferably nanoparticulate eplerenone and a cellulosic excipient, in manufacture of a medicament useful in treatment or prophylaxis of aldosterone-mediated conditions or disorders.

### Process for preparing nanoparticulate eplerenone

The present invention also is directed to processes for preparing nanoparticulate eplerenone and compositions thereof. The first step in preparing a nanoparticulate eplerenone composition is usually to reduce eplerenone to a desired nanoparticulate size range by a suitable process such as is disclosed in any of the patents and publications listed below and incorporated herein by reference.
U.S. Patent No. 4,826,689 to Violanto & Fischer.
Above-cited U.S. Patent No. 5,145,684.
U.S. Patent No. 5,298,262 to Na & Rajagopalan.
U.S. Patent No. 5,302,401 to Liversidge *et al*.
U.S. Patent No. 5,336,507 to Na & Rajagopalan.
U.S. Patent No. 5,340,564 to Illig & Sarpotdar.
U.S. Patent No. 5,346,702 to Na & Rajagopalan.
U.S. Patent No. 5,352,459 to Hollister *et al*.
U.S. Patent No. 5,354,560 to Lovrecich.
Above-cited U.S. Patent No. 5,384,124.
U.S. Patent No. 5,429,824 to June.
U.S. Patent No. 5,503,723 to Ruddy *et al*.
U.S. Patent No. 5,510,118 to Bosch *et al*.
U.S. Patent No. 5,518,187 to Bruno *et al*.
U.S. Patent No. 5,518,738 to Eickhoff *et al*.
U.S. Patent No. 5,534,270 to De Castro.
U.S. Patent No. 5,536,508 to Canal *et al*.
U.S. Patent No. 5,552,160 to Liversidge *et al*.
U.S. Patent No. 5,560,931 to Eickhoff *et al*.
U.S. Patent No. 5,560,932 to Bagchi *et al*.
U.S. Patent No. 5,565,188 to Wong *et al*.
U.S. Patent No. 5,569,448 to Wong *et al*.
U.S. Patent No. 5,571,536 to Eickhoff *et al*.
U.S. Patent No. 5,573,783 to Desieno & Stetsko.
U.S. Patent No. 5,580,579 to Ruddy *et al*.
U.S. Patent No. 5,585,108 to Ruddy *et al*.
U.S. Patent No. 5,587,143 to Wong.
U.S. Patent No. 5,591,456 to Franson *et al*.
U.S. Patent No. 5,622,938 to Wong.
U.S. Patent No. 5,662,883 to Bagchi *et al*.
U.S. Patent No. 5,665,331 to Bagchi *et al*.
U.S. Patent No. 5,718,919 to Ruddy *et al*.
U.S. Patent No. 5,747,001 to Wiedmann *et al*.
Above-cited International Patent Publication No. WO 93/25190.
International Patent Publication No. WO 96/24336.
International Patent Publication No. WO 98/35666.

The eplerenone is obtained commercially and/or prepared by techniques known in the art in a conventional coarse form. It is preferred, but not essential, that the D₉₀ particle size of the coarse eplerenone be less than about 100 µm as determined by sieve analysis. If the initial particle size of the eplerenone is greater than about 100 µm, it is preferred that the particles be reduced in size to less than 100 µm using a conventional milling method such as air jet or fragmentation milling.

In an illustrative process, the coarse eplerenone is added to a liquid medium in which it is essentially insoluble to form a premix suspension. The concentration of the eplerenone in the liquid medium can vary from about 0.1 % to about 60%, and preferably is about 5% to about 30%, by weight. The apparent viscosity of the premix suspension is preferably less than about 1000 cP.

The premix is subjected to mechanical means to reduce the D₉₀ particle size of the eplerenone to less than about 15 µm. It is preferred that the premix be used directly (*i*.*e*., without a prior dispersion step) when a ball mill is used for attrition. Alternatively, the eplerenone can first be dispersed in the liquid medium with suitable agitation, *e.g.*, using a roller mill or a Cowles type mixer, until a homogeneous dispersion is observed in which there are no large agglomerates visible to the naked eye. It is preferred that the premix be subjected to such a dispersion step when a recirculating media mill is used for attrition.

The particles can be milled in presence of a surface modifying agent, for example a polymer or wetting agent. Alternatively, the particles can be contacted with a surface modifying agent after attrition. The surface modifying agent can reduce agglomeration of the particles, and have other benefits.

The mechanical means applied to reduce the particle size of eplerenone can conveniently take the form of a dispersion mill. Suitable dispersion mills include a ball mill, an attritor mill, a vibratory mill, and media mills such as a sand mill and a bead mill. A media mill is preferred due to the relatively short milling time required to provide the desired reduction in particle size. For media milling, the apparent viscosity of the premix preferably is about 100 to about 1000 cP. For ball milling, the apparent viscosity of the premix preferably is about 1 to about 100 cP. Such ranges tend to afford an optimal balance between efficient particle size reduction and media erosion.

The milling time can vary widely and depends primarily upon the particular mechanical means and processing conditions selected. For ball mills, processing times of up to five days or longer may be required. On the other hand, processing times of less than 1 day (residence times of one minute to several hours) can provide the desired results using a high shear media mill.

The particles should be reduced in size at a temperature that does not significantly degrade the eplerenone. Processing temperatures of less than about 30-40°C are ordinarily preferred. If desired, the processing equipment can be cooled with conventional cooling equipment. The method is conveniently carried out at ambient temperature and at processing pressures that are safe and effective for the milling process. For example, ambient processing pressures are typical of ball mills, attritor mills and vibratory mills. Control of the temperature can be achieved by jacketing or immersion of the milling chamber in ice water. Processing pressures from about 0.07 to about 3.5 kg/cm² are contemplated, with pressures of about 0.7 to 1.4 kg/cm² being typical.

After milling is completed, the grinding medium is separated from milled nanoparticulate product (in either a dry or liquid dispersion form) using conventional separation techniques, such as filtration, sieving through a mesh screen or the like.

### Grinding media

A grinding medium for the particle size reduction step can be selected from rigid media, preferably substantially spherical in form, and preferably having an average diameter of less than about 3 mm and, more preferably, less than about I mm. Such media desirably can generate the desired nanoparticles with relatively short processing times and imparting relatively little wear to the milling equipment. The selection of material for the grinding media is not believed to be critical. Zirconium oxide, such as 95% ZrO₂ stabilized with magnesia, zirconium silicate, and glass grinding media provide particles having levels of contamination which arc acceptably low for preparation of pharmaceutical compositions. However, other media, such as stainless steel, titanium dioxide, alumina, and 95% ZrO₂ stabilized with yttrium, are useful. Preferred media have a density greater than about 3 g/cm³.

Alternatively, the grinding medium can comprise particles, preferably substantially spherical in shape, *e*.*g*., beads, consisting essentially of a polymeric resin, or comprising a core having a coating of a polymeric resin adhered thereon. In general, polymeric resins suitable for use herein are chemically and physically inert, substantially free of metals, solvents and monomers, and of sufficient hardness and friability to avoid being chipped or crushed during grinding. Suitable polymeric resins include cross-linked polystyrenes, such as polystyrene cross-linked with divinylbenzene, styrene copolymers, polycarbonates, polyacetals such as Delrin™ vinyl chloride polymers and copolymers, polyurethanes, polyamides, poly(tetrafluoroethylenes) such as Teflon™, other fluoropolymers, high density polyethylenes, polypropylenes, cellulose ethers and esters such as cellulose acetate, polyhydroxymethacrylate, polyhydroxyethyl acrylate, silicone-containing polymers such as polysiloxanes, etc. The polymer can be biodegradable. Illustrative biodegradable polymers include polylactides, polyglycolides, copolymers of lactides and glycolides, polyanhydrides, poly(hydroxyethyl methacrylate), poly(iminocarbonates), poly(N-acylhydroxyproline)esters, poly(N-palmitoyl hydroxyproline) esters, copolymers of ethylene and vinyl acetate, poly(orthoesters), poly(caprolactones) and poly(phosphazenes). In the case of biodegradable polymers, contamination from the grinding medium itself advantageously can metabolize *in vivo* into biologically acceptable products which can be eliminated from the body.

The polymeric resin can have a density from 0.8 to 3.0 g/cm³. Higher density resins are preferred as it is believed that these provide more efficient particle size reduction.

Suitable grinding media range in particle size from about 0.1 to about 3 mm. For fine grinding, the particle size is preferably about 0.2 to about 2 mm, more preferably about 0.25 to about 1 mm.

In a particularly preferred method, the eplerenone is prepared in the form of particles smaller than 1 µm by grinding in the presence of a grinding medium having a mean particle size of less than about 75 µm.

In grinding beads having a polymeric resin deposited on a core, the core material is preferably one known to be useful itself as a grinding medium. Suitable core materials therefore include zirconium oxides (such as 95% zirconium oxide stabilized with magnesia or yttrium), zirconium silicate, glass, stainless steel, titanium dioxide, alumina, ferrite and the like. Preferred core materials have a density greater than about 2.5 g/cm³. The selection of high density core materials is believed to facilitate efficient particle size reduction.

Useful thickness of the polymer coating on the core is about 1 to about 500 µm, although other thicknesses outside this range can be useful in some applications. The thickness of the polymer coating preferably is less than the diameter of the core.

The cores can be coated with the polymeric resin by techniques known in the art. Suitable techniques include spray coating, fluidized bed coating and melt coating. Adhesion-promoting or tie layers can optionally be provided to improve adhesion between the core material and the resin coating. Adhesion can also be enhanced by treating the core material to procedures such as roughening of the core surface, corona discharge treatment and the like.

### Continuous grinding

In a preferred grinding process, the nanoparticles are made continuously rather than in a batch mode. An illustrative continuous process comprises the steps of continuously introducing eplerenone and a rigid grinding medium into a milling chamber, contacting the eplerenone with the grinding medium while in the chamber to reduce the particle size of the eplerenone, continuously removing the eplerenone and the grinding medium from the milling chamber, and thereafter separating the nanoparticulate eplerenone from the grinding medium, for example using conventional separation techniques such as by simple filtration, sieving through a mesh filter or screen, or the like. Other separation techniques such as centrifugation may also be employed.

In a preferred embodiment, the eplerenone and grinding medium are recirculated through the milling chamber. Examples of suitable means to effect such recirculation include conventional pumps such as peristaltic pumps, diaphragm pumps, piston pumps, centrifugal pumps and other positive displacement pumps which do not use sufficiently close tolerances to damage the grinding medium. Peristaltic pumps are generally preferred.

Another variation of the continuous process includes use of mixed media sizes. For example, a larger medium can be employed in a conventional manner, this larger medium being restricted to the milling chamber. A smaller grinding medium can be continuously recirculated through the system and permitted to pass through the agitated bed in the milling chamber. The smaller medium is preferably about 1 to about 300 µm in mean particle size and the larger medium is preferably about 300 to about 1000 µm in mean particle size.

### Eplerenone particle size

Particle size of eplerenone can be measured by conventional techniques known to those skilled in the art, such as sedimentation field flow fractionation, photon correlation spectroscopy, or disk centrifugation. When photon correlation spectroscopy (PCS) is used as the method of particle sizing the average particle diameter is the Z-average particle diameter known to those skilled in the art.

Eplerenone prepared according to processes of the invention, and present in compositions of the invention, has a D₉₀ particle size as defined hereinabove of less than about 15 µm, preferably less than about 10 µm and more preferably less than about 5 µm, for example about 0.01 to about 1 µm. In an especially preferred embodiment the D₉₀ particle size is about 100 to about 800 nm, for example about 100 to about 400 nm, or about 500 to about 800 nm.

It is preferred that at least 95% and, more preferably, at least 99% by weight of the particles have a particle size less than about 15 µm. In particularly preferred embodiments, essentially all of the particles have a size less than about 15 µm.

### Process for preparing nanoparticulate eplerenone compositions

Nanoparticulate eplerenone prepared as described above can be blended, for example in a high shear mixer granulator, planetary mixer, a twin-shell blender or sigma mixer, with one or more excipients. Typically, the nanoparticulate eplerenone is blended with one or more diluent(s), disintegrant(s), binding agent(s) and, optionally, wetting agent(s) in this step. In one embodiment, the nanoparticulate eplerenone is blended with lactose, microcrystalline cellulose, hydroxypropyl methylcellulose and, optionally, sodium lauryl sulfate in the blending step. Blending times as short as three minutes can provide a dry powder mixture having a sufficiently uniform distribution of nanoparticulate eplerenone.

It is possible to add all or a portion of one or more of the excipients in a later step. For example, where microcrystalline cellulose is employed as a diluent and/or disintegrant, addition of a portion of the microcrystalline cellulose during this blending step and addition of the remaining portion after granulation and/or drying steps discussed below can increase hardness and/or decrease friability of tablets produced from the resulting granulation. In this situation, preferably about 40% to about 50% of the microcrystalline cellulose is added intragranularly (before granulation) and about 50% to about 60% of the microcrystalline cellulose is added extragranularly (after granulation).

A preferred process involves wet granulation. According to this process, water is added to the dry powder mixture after blending as described above, and the mixture is blended for an additional period of time to granulate the mixture. The water can be added to the dry powder mixture at once, gradually over a period of time, or in several portions over a period of time. The water preferably is added gradually over a period of time, preferably over at least about 3 to about 5 minutes. An additional period of mixing, generally at least about 1 to about 3 minutes, after the water addition is complete, appears to ensure uniform distribution of the water in the mixture and results in a suitable wet granulated mixture.

It is generally preferred that the wet granulated mixture comprise about 25% to about 45% water by weight. Although a higher or lower water content can be acceptable for certain formulations, a lower water content can reduce effectiveness of the granulation step in producing granules having the desired compressibility and flowability properties, whereas a higher water content can cause an increase in granule size.

The wet granulated mixture is then dried, for example, in an oven or a fluidized bed dryer, preferably a fluidized bed drier. If desired, the wet granulated mixture can be wet milled, extruded and/or spheronized prior to drying, although wet milling is preferred. For the drying process, conditions such as inlet air temperature and drying time are adjusted to achieve the desired moisture content for the dried granulated mixture. Increasing moisture content of the dried granulated mixture from about 2% to about 4% can be found to decrease initial tablet hardness.

To the extent necessary, the dry granules are then reduced in size in preparation for compression. Conventional particle size reduction equipment such as oscillators or Fitz mills can be employed.

The dry granules are then placed in a suitable blender such as a twin-shell blender and a lubricant, anti-adherent agent and/or any additional excipients are added. Although blending times depend in part upon the process equipment used, blending times of at least about 5 to about 25 minutes are generally preferred.

In a preferred embodiment, talc as an anti-adherent agent and a remaining portion of microcrystalline cellulose as a diluent/disintegrant are added to the granules and the mixture is blended for an additional period of time, preferably a period of time sufficient to achieve a blend uniformity of about 6% or less, expressed as relative standard deviation value. Magnesium stearate is then added as a lubricant to the mixture and the mixture is blended for an additional period of time. Addition of a portion of the microcrystalline cellulose after granulation and drying can materially increase tablet hardness. Increasing the amount of magnesium stearate added after granulation and drying can decrease tablet hardness and increase friability and disintegration time.

The mixture resulting from the final blending step above is then compressed into tablets of desired size, shape, weight and hardness using appropriately sized tooling. Alternatively, this mixture can be encapsulated to form capsules of desired size and shape. Conventional compression and encapsulation techniques known to those of ordinary skill in the art can be employed. Where coated tablets are desired, conventional coating techniques known to those of ordinary skill in the art can be employed.

### EXAMPLES

The following examples illustrate aspects of the present invention but should not be construed as limitations. Symbols and conventions used in these examples are consistent with those used in the contemporary pharmaceutical literature. Unless otherwise stated, (i) all percentages recited in these examples are by weight based on total composition weight, (ii) total composition weight for capsules is the total capsule fill weight and does not include the weight of the capsule itself, and (iii) total composition weight for coated tablets does not include the weight of the coating, which typically represents about 3% of the total composition weight before coating.

### Example 1: 25 mg dose immediate-release tablet

A 25 mg dose immediate-release tablet (tablet diameter 5.25 mm) is prepared by a process as described hereinabove and has the following composition:

**Table 1**

| **ingredient** | **weight %** | **mg/tablet** |
|---|---|---|
| nanoparticulate eplerenone | 29.41 | 25.00 |
| lactose monohydrate (#310, NF) | 42.00 | 35.70 |
| microcrystalline cellulose (NF, Avicel™ PH101) | 18.09 | 15.38 |
| intragranular | 7.50 | |
| extragranular | 10.59 | |
| croscarmellose sodium (NF, Ac-Di-Sol™) | 5.00 | 4.25 |
| HPMC (#2910, USP, Pharmacoat™ 603) | 3.00 | 2.55 |
| sodium lauryl sulfate (NF) | 1.00 | 0.85 |
| talc (USP) | 1.00 | 0.85 |
| magnesium stearate (NF) | 0.50 | 0.42 |
| Total | 100 | 85 |
| Opadry™ White YS-1-18027A | 3.00 | 2.55 |

### Example 2: 50 mg dose immediate-release tablet

A 50 mg dose immediate-release tablet (tablet diameter 6.75 mm) is prepared by a process as described hereinabove and has the following composition:

**Table 2**

| **ingredient** | **weight %** | **mg/tablet** |
|---|---|---|
| nanoparticulate eplerenone | 29.41 | 50.00 |
| lactose monohydrate (#310, NF) | 42.00 | 71.40 |
| microcrystalline cellulose (NF, Avicel™ PH101) | 18.09 | 30.75 |
| intragranular | 7.50 | |
| extragranular | 10.59 | |
| croscarmellose sodium (NF, Ac-Di-Sol™) | 5.00 | 8.50 |
| HPMC (#2910, USP, Pharmacoat™ 603) | 3.00 | 5.10 |
| sodium lauryl sulfate (NF) | 1.00 | 1.70 |
| talc (USP) | 1.00 | 1.70 |
| magnesium stearate (NF) | 0.50 | 0.85 |
| Total | 100 | 170 |
| Opadry™ White YS-1-18027A | 3.00 | 5.10 |

### Example 3: 100 mg dose immediate-release tablet

A 100 mg dose immediate-release tablet formulation (tablet diameter 9 mm) is prepared by a process as described hereinabove and has the following composition:

**Table 3**

| **ingredient** | **weight %** | **mg/tablet** |
|---|---|---|
| nanoparticulate eplerenone | 29.41 | 100.00 |
| lactose monohydrate (#310, NF) | 42.00 | 142.80 |
| microcrystalline cellulose (NF, Avicel™ PH101) | 18.09 | 61.50 |
| intragranular | 7.50 | |
| extragranular | 10.59 | |
| croscarmellose sodium (NF, Ac-Di-Sol™) | 5.00 | 17.00 |
| HPMC (#2910, USP, Pharmacoat™ 603) | 3.00 | 10.20 |
| sodium lauryl sulfate (NF) | 1.00 | 3.40 |
| talc (USP) | 1.00 | 3.40 |
| magnesium stearate (NF) | 0.50 | 1.70 |
| Total | 100 | 340 |
| Opadry™ White YS-1-18027A | 3.00 | 10.20 |

### Example 4: 10 mg dose immediate-release capsule

A 10 mg dose immediate-release capsule formulation is prepared by a process as described hereinabove and has the following composition:

**Table 4**

| **ingredient** | **mg/capsule** | **kg/batch** |
|---|---|---|
| nanoparticulate eplerenone | 10.0 | 1.00 |
| lactose, hydrous NF | 306.8 | 30.68 |
| microcrystalline cellulose, NF | 60.0 | 6.00 |
| talc, USP | 10.0 | 1.00 |
| croscarmellose sodium, NF | 8.0 | 0.80 |
| sodium lauryl sulfate, NF | 2.0 | 0.20 |
| colloidal silicon dioxide, NF | 2.0 | 0.20 |
| magnesium stearate, NF | 1.2 | 0.12 |
| total capsule fill weight | 400.0 | 40.00 |
| hard gelatin capsule, size #0, white opaque | 1 capsule | 100,000 capsules |

### Example 5: 25 mg dose immediate-release capsule

A 25 mg dose immediate-release capsule formulation is prepared by a process as described hereinabove and has the following composition:

**Table 5**

| **ingredient** | **mg/capsule** | **kg/batch** |
|---|---|---|
| nanoparticulate eplerenone | 25.0 | 2.50 |
| lactose, hydrous NF | 294.1 | 29.41 |
| microcrystalline cellulose, NF | 57.7 | 5.77 |
| talc, USP | 10.0 | 1.00 |
| croscarmellose sodium, NF | 8.0 | 0.80 |
| sodium lauryl sulfate, NF | 2.0 | 0.20 |
| colloidal silicon dioxide, NF | 2.0 | 0.20 |
| magnesium stearate, NF | 1.2 | 0.12 |
| total capsule fill weight | 400.0 | 40.00 |
| hard gelatin capsule, size #0, white opaque | 1 capsule | 100,000 capsules |

### Example 6: 50 mg dose immediate-release capsule

A 50 mg dose immediate-release capsule formulation is prepared by a process as described hereinabove and has the following composition:

**Table 6**

| **ingredient** | **mg/capsule** | **kg/batch** |
|---|---|---|
| nanoparticulate eplerenone | 50.0 | 5.00 |
| lactose, hydrous NF | 273.2 | 27.32 |
| microcrystalline cellulose, NF | 53.6 | 5.36 |
| talc, USP | 10.0 | 1.00 |
| croscarmellose sodium, NF | 8.0 | 0.80 |
| sodium lauryl sulfate, NF | 2.0 | 0.20 |
| colloidal silicon dioxide, NF | 2.0 | 0.20 |
| magnesium stearate, NF | 1.2 | 0.12 |
| total capsule fill weight | 400.0 | 40.00 |
| hard gelatin capsule, size #0, white opaque | 1 capsule | 100,000 capsules |

### Example 7: 100 mg dose immediate-release capsule

A 100 mg dose immediate-release capsule formulation is prepared by a process as described hereinabove and has the following composition:

**Table 7**

| **ingredient** | **mg/capsule** | **kg/batch** |
|---|---|---|
| nanoparticulate eplerenone | 100.0 | 10.00 |
| lactose, hydrous NF | 231.4 | 23.14 |
| microcrystalline cellulose, NF | 45.4 | 4.54 |
| talc, USP | 10.0 | 1.00 |
| croscarmellose sodium, NF | 8.0 | 0.80 |
| sodium lauryl sulfate, NF | 2.0 | 0.20 |
| colloidal silicon dioxide, NF | 2.0 | 0.20 |
| magnesium stearate, NF | 1.2 | 0.12 |
| total capsule fill weight | 400.0 | 40.00 |
| hard gelatin capsule, size #0, white opaque | 1 capsule | 100,000 capsules |

### Example 8: 200 mg dose immediate-release capsule

A 200 mg dose immediate-release capsule formulation is prepared by a process as described hereinabove and has the following composition:

**Table 8**

| **ingredient** | **mg/capsule** | **kg/batch** |
|---|---|---|
| nanoparticulate eplerenone | 200.0 | 20.00 |
| lactose, hydrous NF | 147.8 | 14.78 |
| microcrystalline cellulose, NF | 29.0 | 2.90 |
| talc, USP | 10.0 | 1.00 |
| croscarmellose sodium, NF | 8.0 | 0.80 |
| sodium lauryl sulfate, NF | 2.0 | 0.20 |
| colloidal silicon dioxide, NF | 2.0 | 0.20 |
| magnesium stearate, NF | 1.2 | 0.12 |
| total capsule fill weight | 400.0 | 40.00 |
| hard gelatin capsule, size #0, white opaque | 1 capsule | 100,000 capsules |

### Example 9: Oral solution

A series of oral solutions is prepared containing 2.5 mg/l eplerenone in a solvent having the following composition: up to 20% by volume ethanol; up to 10% by volume propylene glycol; 10% to 70% by volume glycerol; and 30% to 70% by volume water.

Another series of oral solutions is prepared containing 2.5 mg/l eplerenone and further comprising ethanol, propylene glycol, PEG 400, glycerol and 70% by weight sorbitol.

Another oral solution is prepared in the following manner. A 15% hydroxypropyl-β-cyclodextrin solution in an amount of 20 ml is added to a bottle containing 100 mg eplerenone. The bottle is then placed in a temperature-controlled water bath/shaker at 65°C and shaken for 20 minutes. The bottle is removed from the water bath and permitted to cool at room temperature for about five minutes. A commercially available apple juice in an amount of 60 ml is added to the mixture in the bottle and the contents of the bottle are gently swirled.

The oral solutions of this example are particularly useful in the treatment of, for example, non-ambulatory patients, pediatric patients and patients that have difficulty taking solid dosage forms such as tablets and capsules.

### Example 10: Immediate-release tablets

Immediate-release tablets containing a 100 mg dose of eplerenone and having the composition set forth in Table 10 are prepared by wet granulation (total batch size of 70 g).

**Table 10**

| **ingredient** | **weight %** |
|---|---|
| nanoparticulate eplerenone | 30.0 |
| lactose, hydrous NF | 25.0 |
| microcrystalline cellulose (NF, Avicel™ PH101) | 37.5 |
| croscarmellose sodium (NF, Ac-Di-Sol™) | 2.0 |
| HPMC (#2910, USP, Pharmacoat™ 603) | 3.0 |
| sodium lauryl sulfate (NF) | 1.0 |
| talc (USP) | 1.0 |
| magnesium stearate (NF) | 0.5 |
| Total | 100 |

### Example 11: Two-hour controlled-release tablets

Controlled-release tablets (tablet weight 333.3 mg; round, standard, concave, 9 mm diameter) containing a 100 mg dose of eplerenone are prepared by a process as described hereinabove and have the composition shown in Table 11.

**Table 11**

| **ingredient** | **weight %** |
|---|---|
| nanoparticulate eplerenone | 30.0 |
| lactose monohydrate | 40.0 |
| microcrystalline cellulose (Avicel™ PH 101) | 19.5 |
| HPMC (Methocel™ K4M Premium) | 6.0 |
| HPMC (Pharmacoat™ 603) | 3.0 |
| talc | 1.0 |
| magnesium stearate | 0.5 |
| total | 100 |

### Example 12: Four-hour controlled-release tablets

Controlled-release tablets (round, standard, concave) containing 50 mg (tablet diameter 6.75 mm), 100 mg (tablet diameter 9 mm) and 150 mg (tablet diameter 10.5 mm) doses of eplerenone are prepared by a process as described hereinabove and have the compositions shown in Table 12.

**Table 12**

| **ingredient** | **weight %** | | |
|---|---|---|---|
| | **50 mg** | **100 mg** | **150 mg** |
| nanoparticulate eplerenone | 30.0 | 30.0 | 30.0 |
| lactose monohydrate | 27.0 | 35.7 | 37.0 |
| microcrystalline cellulose (Avicel™ PH 101) | 13.5 | 17.8 | 18.5 |
| HPMC (Methocel™ K4M Premium) | 25.0 | 12.0 | 10.0 |
| HPMC (Pharmacoat™ 603) | 3.0 | 3.0 | 3.0 |
| talc | 1.0 | 1.0 | 1.0 |
| magnesium stearate | 0.5 | 0.5 | 0.5 |
| total | 100 | 100 | 100 |

### Example 13: Six-hour controlled-release tablets

Controlled-release tablets (tablet weight 333.3 mg; round, standard, concave, 9 mm diameter) containing a 100 mg dose of eplerenone are prepared by a process as described hereinabove and have the composition shown in Table 13.

**Table 13**

| **ingredient** | **weight %** |
|---|---|
| nanoparticulate eplerenone | 30.0 |
| lactose monohydrate | 30.5 |
| microcrystalline cellulose (Avicel™ PH 101) | 15.0 |
| HPMC (Methocel® K4M Premium) | 20.0 |
| HPMC (Pharmacoat™ 603) | 3.0 |
| talc | 1.0 |
| magnesium stearate | 0.5 |
| total | 100 |

### Example 14: Tablets

Tablets containing a 100 mg dose or a 200 mg dose of eplerenone and having the compositions set forth in Table 14 below are prepared by wet granulation (total batch size of 1 kg).

**Table 14**

| **ingredient** | **weight fraction of tablet (%)** | | | | | |
|---|---|---|---|---|---|---|
| | A | B | C | D | E | F |
| nanoparticulate eplerenone | 30 | 30 | 30 | 30 | 30 | 30 |
| lactose monohydrate | 10 | 40 | 10 | 40 | 25 | 25 |
| microcrystalline cellulose (Avicel™ PH 101) | 50.5 | 20.5 | 35.5 | 5.5 | 28 | 28 |
| HPMC (Methocel™ K4M Premium) | 5 | 5 | 20 | 20 | 12.5 | 12.5 |
| HPMC (Pharmacoat™ 603) | 3 | 3 | 3 | 3 | 3 | 3 |
| talc | 1 | 1 | 1 | 1 | 1 | 1 |
| magnesium stearate | 0.5 | 0.5 | 0.5 | 0.5 | 0.5 | 0.5 |
| total | 100 | 100 | 100 | 100 | 100 | 100 |

### Example 15: Controlled-release tablets

Controlled-release ("CR") tablets containing a 100 mg dose of eplerenone and having the compositions set forth in Table 15 below are prepared by wet granulation (total batch size of 70 g).

**Table 15**

| **ingredient** | **weight fraction of tablet (%)** | | |
|---|---|---|---|
| | **2-hour CR** | **4-hour CR** | **6-hour CR** |
| nanoparticulate eplerenone | 30 | 30 | 30 |
| lactose monohydrate | 40 | 36 | 30.5 |
| microcrystalline cellulose (Avicel™ PH 101) | 17.5 | 15.5 | 15 |
| HPMC (Methocel™ K4M Premium) | 8 | 14 | 20 |
| HPMC (Pharmacoat™ 603) | 3 | 3 | 3 |
| talc | 1 | 1 | I |
| magnesium stearate | 0.5 | 0.5 | 0.5 |
| total | 100 | 100 | 100 |

### Example 16: Controlled-release tablets

Two-hour, four-hour and six-hour controlled-release tablets containing a 100 mg dose of eplerenone are prepared by wet granulation in a scaled-up process (total batch sizes of 2 kg and 10 kg). The tablets have the same compositions as set forth in Table 15 above, except that the two-hour and four-hour compositions have high molecular weight HPMC (Methocel™ K4M Premium) weight fractions of 6% and 12%, respectively, and microcrystalline cellulose weight fractions of 19.5% and 17.5%, respectively.

## Claims

1. Eplerenone particles wherein 90% by weight of the particles are smaller than about 15 µm.

2. The eplerenone particles of Claim 1 wherein 90% by weight of the particles are smaller than about 1 µm.

3. A pharmaceutical composition comprising (a) the eplerenone particles of Claim 1 in an amount of about 10 mg to about 1000 mg and (b) one or more pharmaceutically acceptable excipients.

4. The composition of Claim 3 that is in the form of an orally deliverable tablet or capsule, wherein the amount of eplerenone is about 25 mg to about 150 mg, and the excipients comprise one or more diluents, one or more disintegrants and one or more binding agents.

5. The composition of Claim 4 wherein the excipients further comprise one or more wetting agents, one or more lubricants and/or one or more anti-adherents.

6. The composition of Claim 3 that is orally deliverable, wherein the amount of eplerenone is about 1% to about 90% by weight of the composition, and wherein the excipients comprise:
(a) one or more diluents in an amount of about 5% to about 99% by weight of the composition, the diluents being selected from the group consisting of lactose including anhydrous lactose and lactose monohydrate, starches including directly compressible starch and hydrolyzed starches, mannitol, sorbitol, xylitol, dextrose, dextrose monohydrate, dibasic calcium phosphate dihydrate, sucrose-based diluents, confectioner's sugar, monobasic calcium sulfate monohydrate, calcium sulfate dihydrate, granular calcium lactate trihydrate, dextrates, inositol, hydrolyzed cereal solids, amylose, celluloses including microcrystalline cellulose, food grade sources of α- and amorphous cellulose and powdered cellulose, calcium carbonate, glycine, bentonite and polyvinylpyrrolidone;
(b) one or more disintegrants in an amount of about 0.5% to about 30% by weight of the composition, the disintegrants being selected from the group consisting of starches including pregelatinized corn starches and sodium starch glycolate, clays, celluloses including purified cellulose, microcrystalline cellulose, methylcellulose, carboxymethylcellulose, sodium carboxymethylcellulose and croscarmellose sodium, alginates, crospovidone and gums including agar, guar, locust bean, karaya, pectin and tragacanth gums;
(c) one or more binding agents in an amount of about 0.5% to about 25% by weight of the composition, the binding agents being selected from the group consisting of acacia, tragacanth, sucrose, gelatin, glucose, starches including pregelatinized starches, celluloses including methylcellulose and sodium carboxymethylcellulose, alginic acid and salts thereof, magnesium aluminum silicate, polyethylene glycol, guar gum, polysaccharide acids, bentonites, polyvinylpyrrolidone, polymethacrylates, hydroxypropylmethylcellulose, hydroxypropylcellulose and ethylcellulose;
(d) optionally one or more wetting agents in an amount of about 0.1% to about 15% by weight of the composition, the wetting agents if present being selected from the group consisting of quaternary ammonium compounds including benzalkonium chloride, benzethonium chloride and cetylpyridinium chloride, dioctyl sodium sulfosuccinate, polyoxyethylene alkylphenyl ethers including nonoxynol 9, nonoxynol 10 and octoxynol 9, poloxamers, polyoxyethylene fatty acid glycerides and oils including polyoxyethylene (8) caprylic/capric mono- and diglycerides, polyoxyethylene (35) castor oil and polyoxyethylene (40) hydrogenated castor oil, polyoxyethylene alkyl ethers including polyoxyethylene (20) cetostearyl ether, polyoxyethylene fatty acid esters including polyoxyethylene (40) stearate, polyoxyethylene sorbitan esters including polysorbate 20 and polysorbate 80, propylene glycol fatty acid esters including propylene glycol laurate, sodium lauryl sulfate, fatty acids and salts thereof including oleic acid, sodium oleate and triethanolamine oleate, glyceryl fatty acid esters including glyceryl monostearate, sorbitan esters including sorbitan monolaurate, sorbitan monooleate, sorbitan monopalmitate and sorbitan monostearate, and tyloxapol;
(e) optionally one or more lubricants in an amount of about 0.1 % to about 10% by weight of the composition, the lubricants if present being selected from the group consisting of glyceryl behapate, stearic acid and salts thereof including magnesium, calcium and sodium stearates, hydrogenated vegetable oils, colloidal silica, talc, waxes, boric acid, sodium benzoate, sodium acetate, sodium fumarate, sodium chloride, DL-leucine, polyethylene glycols, sodium oleate, sodium lauryl sulfate and magnesium lauryl sulfate; and
(f) optionally one or more anti-adherents in an amount of about 0.25% to about 10% by weight of the composition, the anti-adherents if present being selected from talc, cornstarch, DL-leucine, sodium lauryl sulfate and metallic stearates.

7. The composition of Claim 3 in the form of an orally deliverable tablet or capsule that, in a standard dissolution assay using a 1% aqueous sodium dodecyl sulfate dissolution medium, releases about 50% of the eplerenone contained therein in 6 hours or less.

8. The composition of Claim 7 that is an immediate-release tablet or capsule comprising about 20 to about 110 mg nanoparticulate eplerenone; about 30 to about 150 mg lactose monohydrate; about 10 to about 70 mg microcrystalline cellulose; about 1 to about 15 mg hydroxypropylmethylcellulose having a viscosity, 2% in water, of about 2 to about 8 cP; optionally about 1 to about 25 mg croscarmellose sodium; optionally about 0.25 to about 5 mg sodium lauryl sulfate; optionally about 0.5 to about 3 mg magnesium stearate; and optionally about 0.5 to about 5 mg talc.

9. The composition of Claim 7 that is a controlled-release tablet or capsule comprising about 25 to about 150 mg nanoparticulate eplerenone; about 12.5 to about 190 mg lactose monohydrate; about 2 to about 100 mg microcrystalline cellulose; about 10 to about 80 mg high molecular weight HPMC having a viscosity, 2% in water, of about 3,500 to about 5,600 cP; about 1 to about 25 mg low molecular weight HPMC having a viscosity, 2% in water, of about 2 to about 8 cP; optionally about 0.1 to about 10 mg magnesium stearate; and optionally about 0.5 to about 15 mg talc.

10. The composition of Claim 3 that provides a therapeutic effect as an aldosterone receptor blocker in a human subject over an interval of about 12 to about 24 hours after oral administration of the composition.

11. A therapeutic method comprising orally administering eplerenone particles, 90% by weight of which are smaller than about 15 µm, in a daily dosage amount of about 10 to about 1000 mg eplerenone, to a subject having an aldosterone-mediated condition or disorder.

12. The method of Claim 11 wherein the condition or disorder is selected from the group consisting of heart failure, hypertension, edema associated with liver insufficiency, post-myocardial infarction, cirrhosis of the liver and accelerated heart rate.

13. The method of Claim 11 wherein the eplerenone particles are formulated with one or more pharmaceutically acceptable excipients in an orally deliverable composition.

14. A method of use of eplerenone particles, 90% by weight of which are smaller than about 15 µm, in manufacture of a medicament for treatment or prophylaxis of an aldosterone-mediated condition or disorder.
